# EUROPEAN PATENT APPLICATION

(11) **EP 4 464 336 A1**
(43) Date of publication of application: **20.11.2024**
(21) Application number: 23740372.0
(22) Date of filing: 12.01.2023
(51) Int. Cl.: A61K 47/64, A61K 9/107, A61K 38/19, A61K 38/20, A61K 47/34, A61P 35/00, A61P 37/04

(54) **CYTOKINE-ENCAPSULATING POLYMERIC MICELLE**

(30) Priority: 12.01.2022 JP 2022002782
(71) Applicant: The University of Tokyo, Bunkyo-ku, Tokyo 113-8654 (JP)
(72) Inventor: CABRAL Horacio, Tokyo 113-8654 (JP); CHEN Pengwen, Tokyo 113-8654 (JP); MIYAZAKI Takuya, Tokyo 113-8654 (JP)
(74) Representative: J A Kemp LLP
(86) International application number: PCT/JP2023/001601
(87) International publication number: WO 2023/136364

(57) **Abstract**

The present invention provides a polymeric complex comprising a cytokine and a block copolymer represented by the following formula (1).

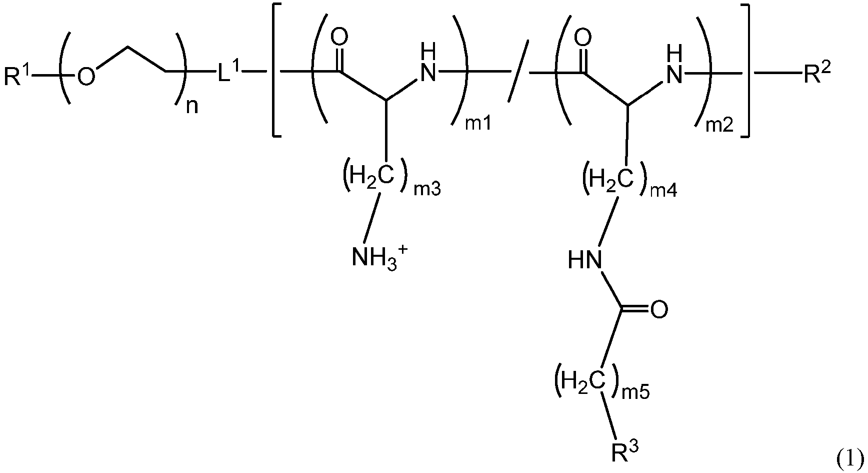

## Description

### Technical Field

The present invention relates to a cytokine-encapsulating polymeric micelle, particularly an interleukin 12-encapsulating micelle, which is configured to achieve improved stability in a severe *in vivo* environment by using a block copolymer. All disclosures of the references cited herein are incorporated herein by reference in their entirety.

### Background Art

Proteins are physiologically active substances found in everywhere in the body, and therefore have been used in the treatment of various intractable diseases including cancers, autoimmune diseases and metabolic disorders. However, when systemically administered alone, proteins undergo enzymatic degradation and/or renal excretion, and further have immunogenicity, so that the biomedical application of proteins requires the development of delivery carriers. For this purpose, efforts have been made to develop protein-PEG conjugates in which a biocompatible polymer, polyethylene glycol) (PEG), is introduced into proteins, whereby the problems associated with proteins can be overcome by suppressed interactions with proteases and/or immunocytes and increased size^{[1-4]}. In actual fact, many protein-PEG conjugates have been approved by the FDA, and their market as protein formulations is worth several billions of dollars^{[5,6]}. However, when proteins are PEGylated, their enzymatic degradation, renal excretion and immunogenicity are suppressed^{[7,8]}, although there arise problems such as protein inactivation caused by irreversible chemical modifications to proteins, and insufficient spatial-temporal regulation of protein functions^{[6,9]}. Thus, efforts have been made to develop delivery carriers which are designed to formulate proteins via reversible chemical bonds, whereby the proteins can be released in a target tissue specific manner while suppressing protein expression in normal tissues^{[10]}.

Stimuli-responsive nanocarriers are designed to detect physiologically active substances in target tissues, whereby proteins can be released in a target tissue specific manner while retaining their activity^{[4,11]}. Among such nanocarriers, core-shell type polymeric micelles formed upon autonomous association between block copolymer and protein can induce protein release in response to external stimuli by introducing environmentally responsive sites into the core-forming chain of the block copolymer^{[4]}. External stimuli to which polymeric micelles can respond may be exemplified by pH. For example, many diseases (e.g., cancers or autoimmune diseases) show lower pH values (pH 6.5 to 7.2) than normal tissues (pH 7.4)^{[12,13]}.

On the other hand, the inventors of the present invention have previously shown that polyion complex (PIC)-type polymeric micelles can be prepared by addition of a PEG-polycation to a protein whose amino groups have been converted into carboxyl groups by a pH-responsive maleic anhydride derivative^{[14-16]}. Micelles of this type encapsulate a protein stably within the core at normal tissue pH (pH 7.4), but the pH-responsive maleic anhydride derivative is cleaved at an acidic pH in target tissues (pH 6.5 to 7.2), thereby successfully releasing the protein.

However, for their medical application, it is important to improve their blood retention and thereby enhance their accumulation into target tissues.

### Prior Art Documents

### Non-patent Documents

[Non-patent Document 1] R. Langer, D. A. Tirrell, Nature 2004, 428, 487-492
[Non-patent Document 2] B. Romberg, W. E. Hennink, G. Storm, Pharm. Res. 2008, 25, 55-71.
[Non-patent Document 3] V. Torchilin, Adv. Drug Deliv. Rev. 2011, 63, 131-135.
[Non-patent Document 4] H. Cabral, K. Miyata, K. Osada, K. Kataoka, Chem. Rev. 2018, 118, 6844-6892.
[Non-patent Document 5] Y Qi, A. Chilkoti, Curr. Opin. Chem. Biol. 2015, 28, 181-193.
[Non-patent Document 6] S. N. S. Alconcel, A. S. Baas, H. D. Maynard, Polym. Chem. 2011, 2, 1442-1448.
[Non-patent Document 7] F. M. Veronese, G. Pasut, Drug Discov. Today 2005, 10, 1451-1458.
[Non-patent Document 8] F. F. Abuchowski, A., McCoy, J. R., Palczuk, N. C., van Es, T., Davis, J. Biol. Chem. 1977, 252, 3582-3586.
[Non-patent Document 9] J. L. Kaar, K. Matyjaszewski, A. J. Russell, C. M. Colina, A. Simakova, B. S. Sumerlin, C. A. Figg, S. L. Baker, AIChE J. 2018, 64, 3230-3245.
[Non-patent Document 10] Y Lu, W. Sun, Z. Gu, J. Control. Release 2014, 194, 1-19.
[Non-patent Document 11] S. Mura, J. Nicolas, P. Couvreur, Nat. Mater. 2013, 12, 991.
[Non-patent Document 12] L. E. Gerweck, K. Seetharaman, Cancer Res. 1996, 56, 1194 LP - 1198.
[Non-patent Document 13] G. Helmlinger, F. Yuan, M. Dellian, R. K. Jain, Nat. Med. 1997, 3, 177-182.
[Non-patent Document 14] Y Lee, T. Ishii, H. Cabral, H. J. Kim, J. H. Seo, N. Nishiyama, H. Oshima, K. Osada, K. Kataoka, Angew. Chemie - Int. Ed. 2009, 48, 5309-5312.
[Non-patent Document 15] Y Lee, T. Ishii, H. J. Kim, N. Nishiyama, Y Hayakawa, K. Itaka, K. Kataoka, Angew. Chemie 2010, 122, 2606-2609.
[Non-patent Document 16] A. Kim, Y Miura, T. Ishii, O. F. Mutaf, N. Nishiyama, H. Cabral, K. Kataoka, Biomacromolecules 2016, 17, 446-453.

### Summary of the Invention

### Problem to be Solved by the Invention

Accordingly, for enhancement of the therapeutic effect provided by therapeutic proteins, it is important to develop micelles which allow increased blood retention and efficient protein release under acidic conditions.

### Means to Solve the Problem

The present invention aimed at increased stability of micelles and efficient release of a protein under acidic conditions by introducing a pH-responsive maleic anhydride derivative into the core-forming chain of a block copolymer to thereby form reversible covalent bonds with amino groups in the protein. Moreover, the present invention aimed at further stabilization of micelles by PIC formation between amino groups in the core-forming chain of the block copolymer and carboxyl groups in the protein. The object of the present invention is to stabilize the structure of micelles by covalent bonding and PIC formation and thereby enhance their blood retention. Moreover, the inventors of the present invention have succeeded in encapsulating interleukin 12 (IL-12) as a protein of interest, and this success led to the completion of the present invention.

Namely, the present invention is as follows.
[1] A polymeric complex comprising a cytokine and a block copolymer represented by the following formula (1): 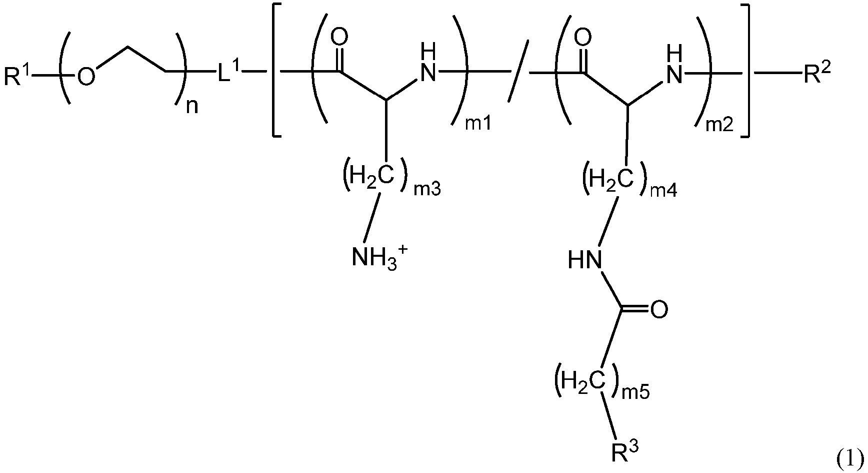
   [wherein R¹ and R² each independently represent a hydrogen atom, or an optionally substituted linear or branched alkyl group containing 1 to 12 carbon atoms, or an azide, an amine, maleimide, a ligand or a labeling agent,
      R³ represents a compound represented by the following formula (I): 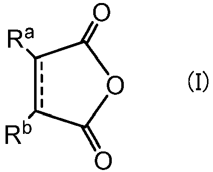
   (wherein R^{a} and R^{b} each independently represent a hydrogen atom, or an optionally substituted alkyl group, an alkenyl group, a cycloalkyl group, an aryl group, an aralkyl group, an acyl group, a heterocyclic group, a heterocyclic alkyl group, a hydroxy group, an alkoxy group or an aryloxy group. Alternatively, R^{a} and R^{b} may be joined with each other to form an aromatic ring or a cycloalkyl ring together with the carbon atoms to which they are attached respectively. The bond between the carbon atoms to which R^{a} and R^{b} are attached respectively may be a single bond or a double bond),
      L¹ represents NH, CO, or a group represented by the following formula (11):

         -(CH₂)ₚ₁-NH- (11)

         (wherein p1 represents an integer of 1 to 6), or
         a group represented by the following formula (12):

            -L^{2a}-(CH₂)_{q1}-L^{3a}- (12)
         (wherein L^{2a} represents OCO, OCONH, NHCO, NHCOO, NHCONH, CONH or COO, L^{3a} represents NH or CO, and q1 represents an integer of 1 to 6),
      m1 and m2 each independently represent an integer of 0 to 500 (provided that the sum of m1 and m2 represents an integer of 10 to 500), m3, m4 and m5 each independently represent an integer of 1 to 5, and n represents an integer of 0 to 500, and
      the symbol "/" means that (m1 + m2) units of the respective monomer units shown on the left and right sides of this symbol may be in any sequence].
[2] The complex according to [1] above, wherein the compound represented by formula (I) is at least one of compounds represented by the following formulae (Ia) to (Ig). 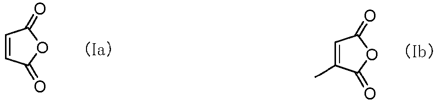 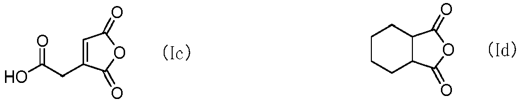 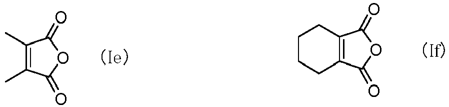 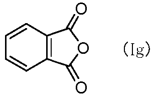
[3] The complex according to [2] above, wherein the compound represented by formula (I) is a compound represented by the following formula (Ia) or (Ib). 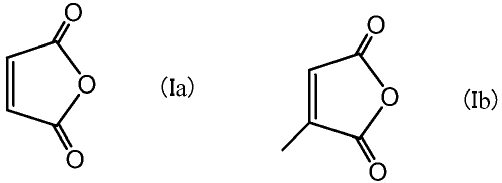
[4] The complex according to [1] above, wherein the block copolymer represented by formula 1 is a block copolymer represented by the following formula (2). 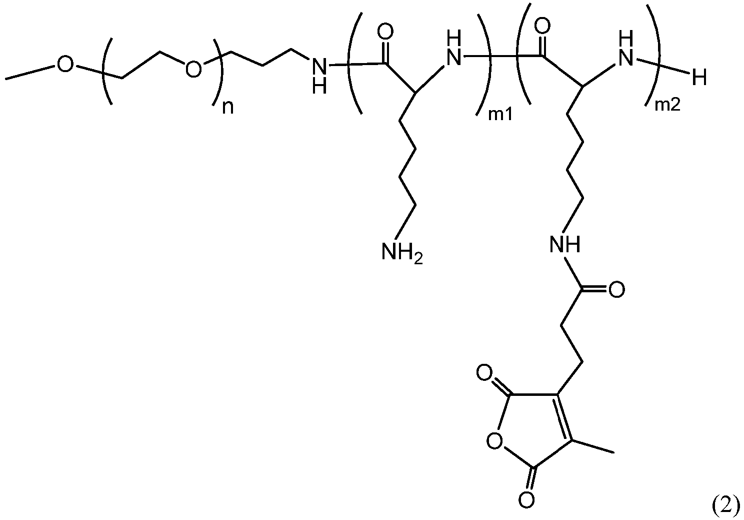
[5] The complex according to [1] above, wherein the cytokine is covalently bonded to the block copolymer represented by formula 1.
[6] The complex according to [5] above, wherein the covalent bond is cleaved in a pH-dependent manner.
[7] The complex according to any one of [1] to [6] above, wherein the cytokine is interleukin 12.
[8] A cytokine delivery device comprising the polymeric complex according to any one of [1] to [7] above for use in cytokine delivery to any site selected from a cell surface site, an intracellular site and an extracellular site.
[9] A cytokine delivery kit comprising a block copolymer represented by the following formula (1) for use in cytokine delivery to any site selected from a cell surface site, an intracellular site and an extracellular site: 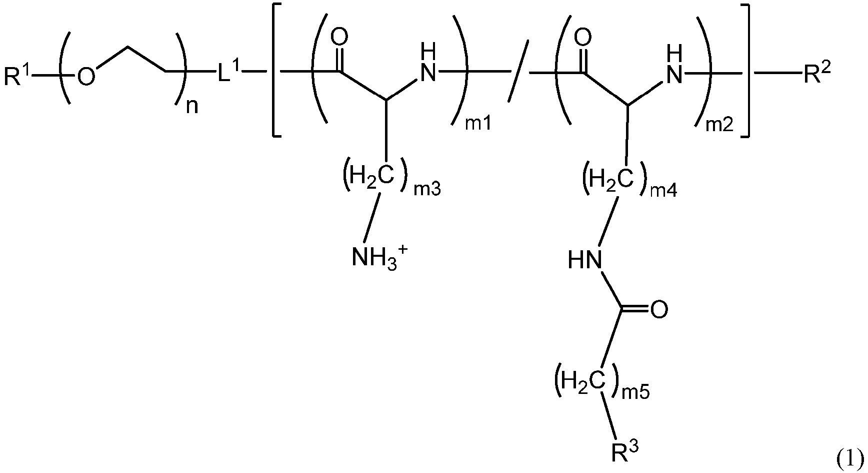
   [wherein R¹ and R² each independently represent a hydrogen atom, or an optionally substituted linear or branched alkyl group containing 1 to 12 carbon atoms, or an azide, an amine, maleimide, a ligand or a labeling agent,
      R³ represents a compound represented by the following formula (I): 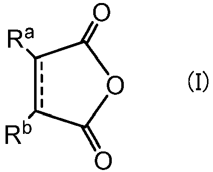
   (wherein R^{a} and R^{b} each independently represent a hydrogen atom, or an optionally substituted alkyl group, an alkenyl group, a cycloalkyl group, an aryl group, an aralkyl group, an acyl group, a heterocyclic group, a heterocyclic alkyl group, a hydroxy group, an alkoxy group or an aryloxy group. Alternatively, R^{a} and R^{b} may be joined with each other to form an aromatic ring or a cycloalkyl ring together with the carbon atoms to which they are attached respectively. The bond between the carbon atoms to which R^{a} and R^{b} are attached respectively may be a single bond or a double bond),
      L¹ represents NH, CO, or a group represented by the following formula (11):

         -(CH₂)ₚ₁-NH- (11)

         (wherein p1 represents an integer of 1 to 6), or
         a group represented by the following formula (12):

            -L^{2a}-(CH₂)_{q1}-L^{3a}- (12)
         (wherein L^{2a} represents OCO, OCONH, NHCO, NHCOO, NHCONH, CONH or COO,
         L^{3a} represents NH or CO, and q1 represents an integer of 1 to 6),
      m1 and m2 each independently represent an integer of 0 to 500 (provided that the sum of m1 and m2 represents an integer of 10 to 500), m3, m4 and m5 each independently represent an integer of 1 to 5, and n represents an integer of 0 to 500, and
      the symbol "/" means that (m1 + m2) units of the respective monomer units shown on the left and right sides of this symbol may be in any sequence].
[10] The kit according to [9] above, wherein the compound represented by formula (I) is at least one of compounds represented by the following formulae (Ia) to (Ig). 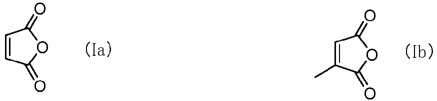 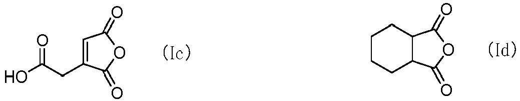 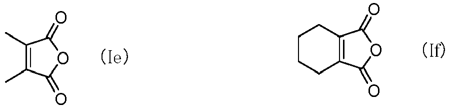 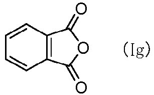
[11] The kit according to [9] above, wherein the compound represented by formula (I) is a compound represented by the following formula (Ia) or (Ib). 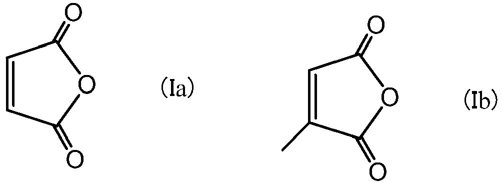
[12] The kit according to [9] above, wherein the block copolymer represented by formula 1 is a block copolymer represented by the following formula (2). 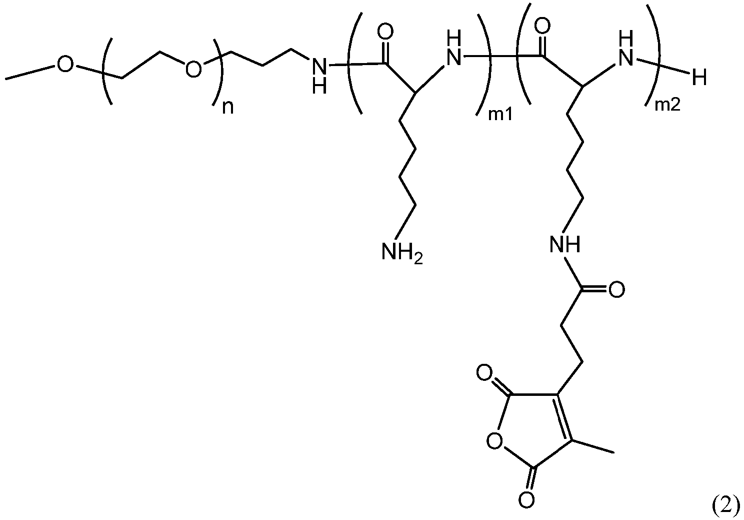
[13] The kit according to any one of [9] to [12] above, wherein the cytokine is interleukin 12.
[14] A pharmaceutical composition comprising the polymeric complex according to any one of [1] to [7] above.
[15] The pharmaceutical composition according to [14] above for use in immunotherapy or antitumor therapy.

### Brief Description of the Drawings

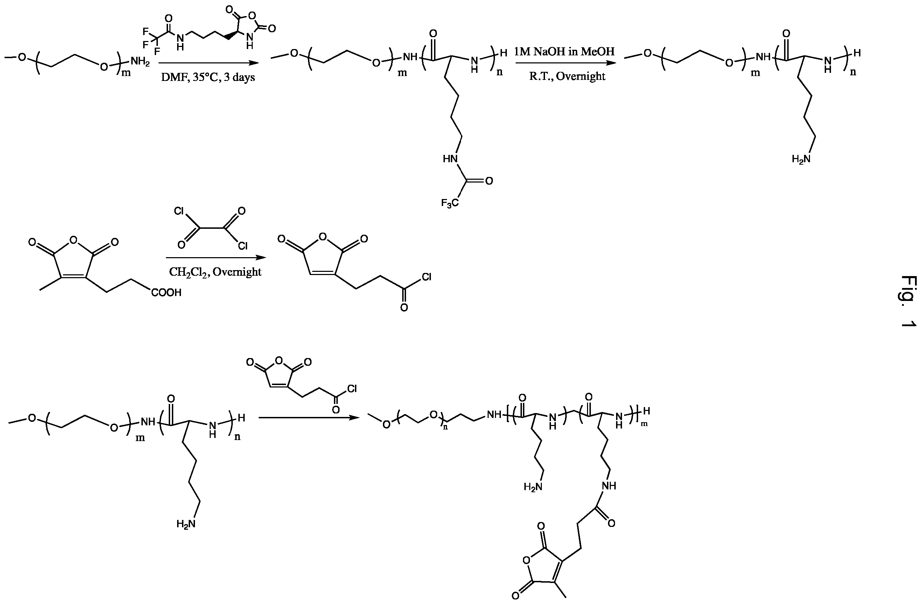

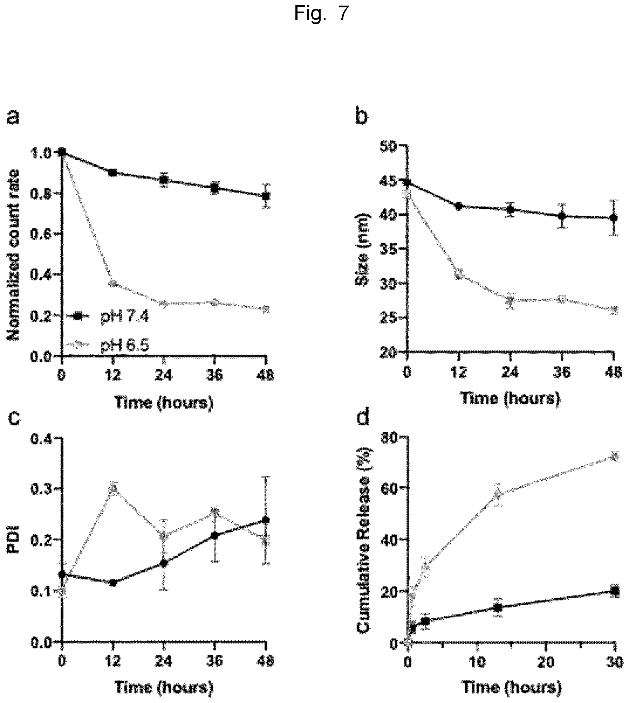

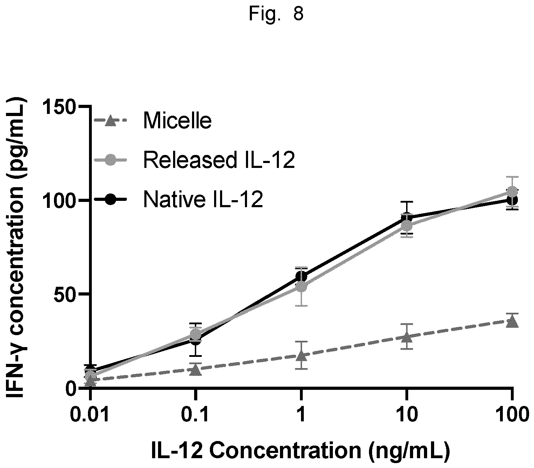

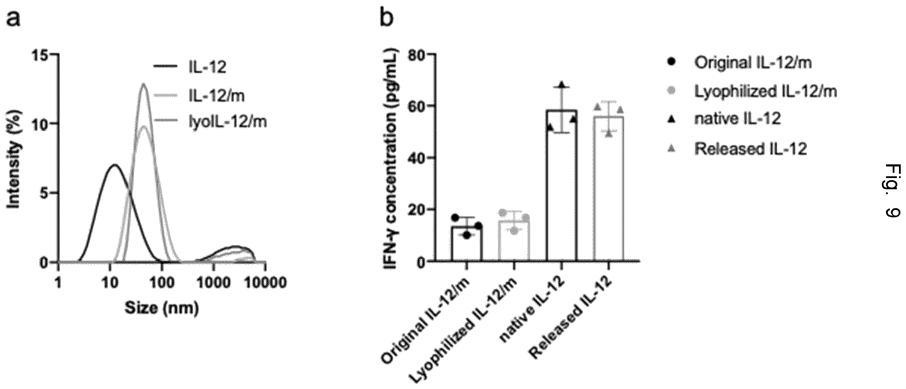

Figure 1 shows the synthesis scheme of PEG-p(Lys-CDM).
   PEG-pLys(TFA) was synthesized by ring-opening polymerization of Lys(TFA)-NCA initiated with MeO-PEG-NH₂, followed by deprotection of the TFA group to obtain PEG-pLys. CDM was reacted with oxalyl chloride to generate CDM-Cl, and CDM was linked to PEG-pLys through reaction between CDM-Cl and lysine amine.
Figure 2 shows the characterization of PEG-pLys(TFA).
   a) ¹H-NMR result obtained for the product dissolved in DMSO. The polymer was determined to have the formula PEG₁₂ₖ-pLys(TFA)₄₀. b) GPC result obtained for PEG₁₂ₖ-pLys(TFA)₄₀ dissolved in DMF. Mw/Mn = 1.029
Figure 3 shows the characterization of PEG-pLys.
   a) ¹H-NMR result obtained for the product dissolved in D₂O. The polymer was determined to have the formula PEG₁₂ₖ-pLys₄₀. b) GPC result obtained for PEG₁₂ₖ-pLys ₄₀ dissolved in PBS.
Figure 4 shows the characterization of PEG-p(Lys-CDM).
   a) ¹H-NMR result obtained for the product dissolved in DMSO. This polymer was determined to have the formula PEG₁₂ₖ-p(Lys₂₀-CDM₂₀). b) GPC result obtained for PEG₁₂ₖ-p(Lys₂₀-CDM₂₀) dissolved in acetate buffer of pH 3.3.
Figure 5 shows how to prepare micelles.
   a) Scheme visualizing the preparation protocol. b) The results of HPLC suggested the success of encapsulation, as indicated by peak shift, and the efficiency of encapsulation was shown by comparison of peak areas. c) ELISA assay was used to determine the efficiency of encapsulation in a system containing non-labeled IL-12. After dilution, the total IL-12 feed to the system is 500 pg/mL, and a red point indicates the result measured for free IL-12 in the system. The efficiency of encapsulation was determined to be about 68%.

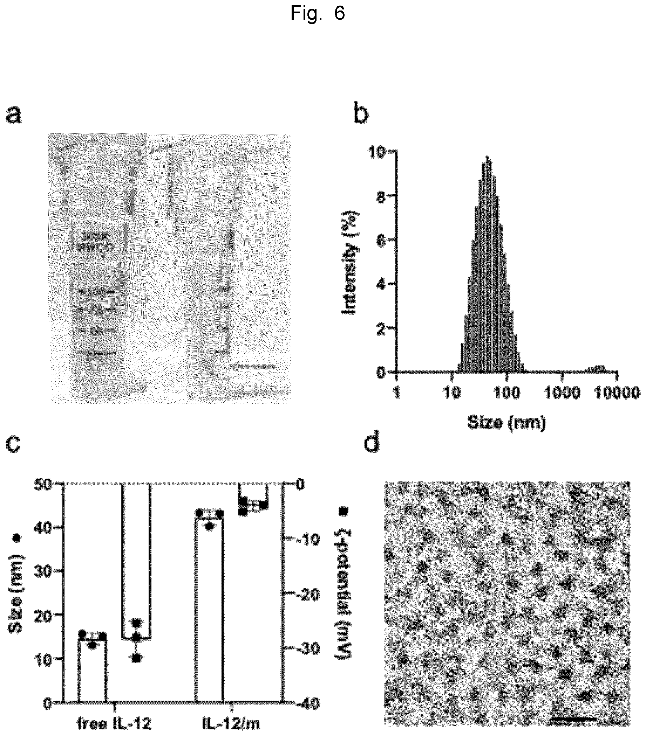

Figure 6 shows the characterization of IL-12/m.
   a) The formation of micelles was visualized with Alexa 647-IL-12 following ultrafiltration through a 300,000 MWCO membrane. b) Representative result of DLS. c) Size and zeta potential of IL-12 and IL-12/m. d) TEM image of IL-12/m. Scale bar = 100 nm
Figure 7 shows the pH-sensitive stability and cargo release of IL-12/m.
   a) Normalized count rates of the solution after different incubation times. The count rates were normalized to the corresponding initial value. b) Average size changes during the incubation time. c) PDI varies depending on the incubation time. d) IL-12 release kinetics at pH 6.5 and pH 7.4. n = 3
Figure 8 shows an *in vitro* assay for testing the biological activity of IL-12/m and released IL-12.
   Spleen cells were treated with samples of different IL-12 equivalent concentrations. Native IL-12 from the same batch was used for reference (black line). n = 4
Figure 9 shows the size distribution and biological activity of lyo-IL-12/m.
   a) The results of DLS indicated that lyo-IL-12/m had the same size distribution as original IL-12/m. b) In an *in vitro* spleen cell assay, lyo-IL-12/m and original IL-12/m (n = 3) were confirmed to have the same biological activity.

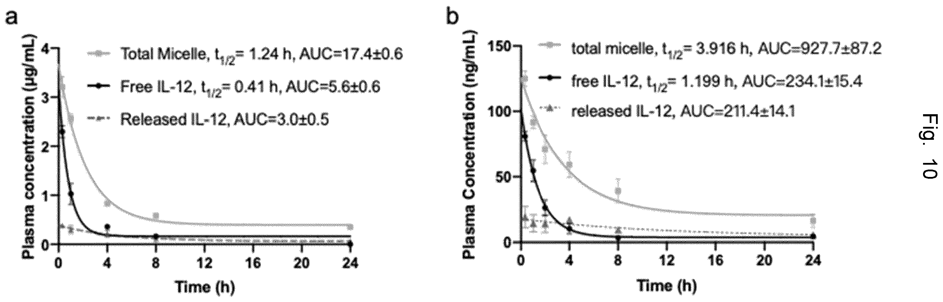

Figure 10 shows the blood circulation of IL-12/m at different injection doses.
   a) 10 µg IL-12 equivalence; b) 1 µg IL-12 equivalence. The half-life was calculated from a monophasic attenuation model.
   n = 3, AUC was calculated using the monophasic attenuation model.

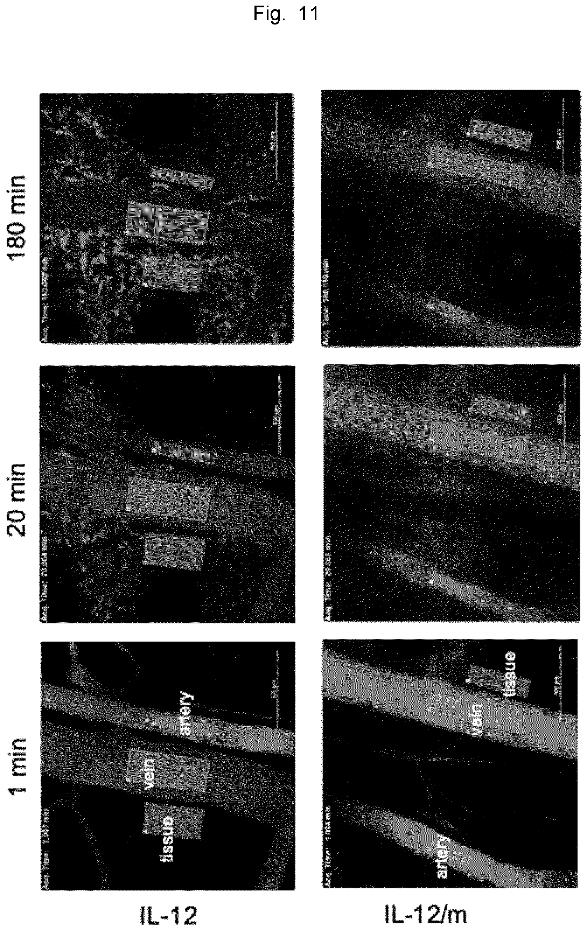

Figure 11 shows the representative results of IVCLM.
   IVCLM occurs in the earlobes of mice intravenously injected with 10 µg of fluorescently labeled IL-12 or equivalent IL-12/m. Clear fluorescence was detectable from the tissue region (blue) at 20 minutes after injection in the IL-12-treated mouse, whereas the fluorescence detected from the tissue in the IL-12/m-treated mouse was minimal. Scale bar = 100 µm.

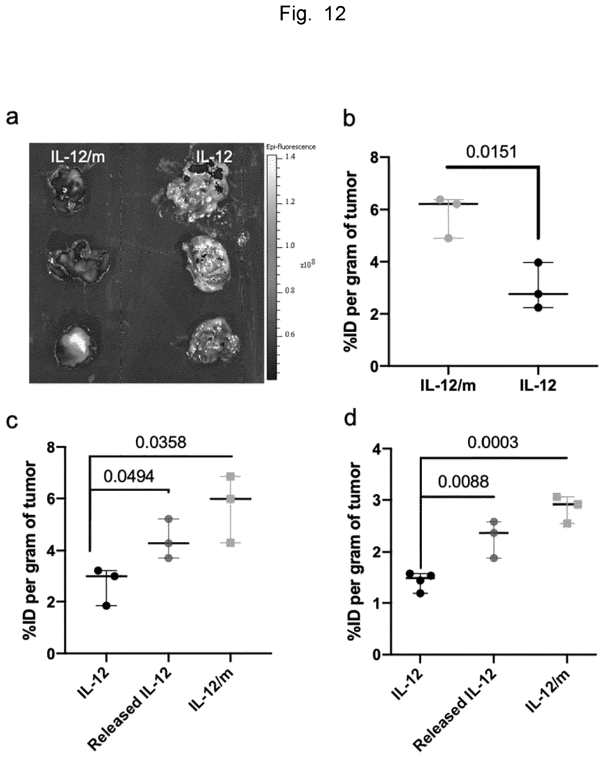

Figure 12 shows the accumulation of IL-12/m in tumor sites at 24 hours after injection.
   a) IVIS image of melanoma after injection of Alexa 647-IL-12 or equivalent micelle (10 µg). b) Fluorescence intensity from melanoma was quantified. c) Data were normalized to the ELISA data measured for homogenates from TNBC tumor injected with 10 µg of non-labeled IL-12 or equivalent micelle. d) ELISA assay on homogenates from melanoma tumor injected with 1 µg of non-labeled IL-12 or equivalent micelle.
   n = 3, p-values were analyzed by unpaired t-test.

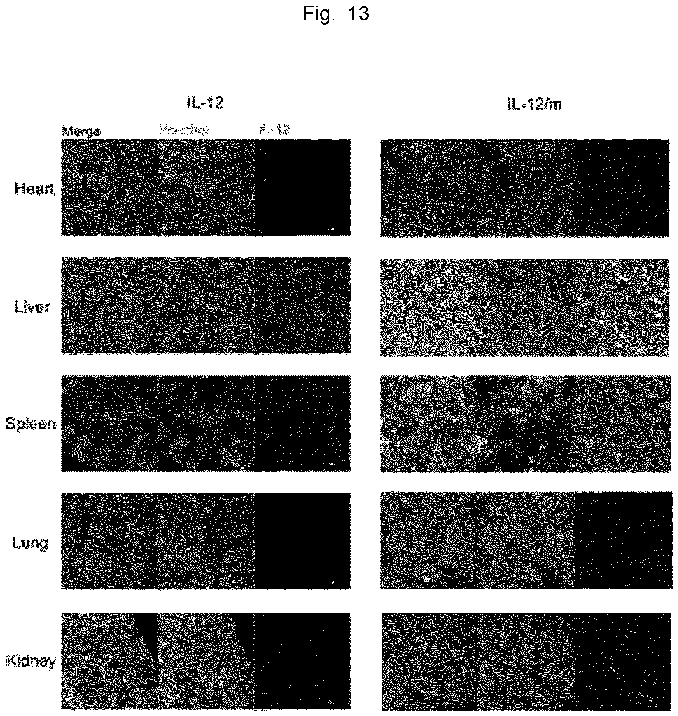

Figure 13 shows the representative IVCLSM images observed for organs at 3 hours after injection.
   Tumor-free mice were injected with 10 µg of fluorescently labeled IL-12 or equivalent IL-12/m. At 2.5 hours after injection, 30 µL of a Hoechst 33342 solution was intravenously injected. At 3 hours after injection, organ samples were collected from the mice and observed under an IVCLSM. Scale bar = 100 µm.

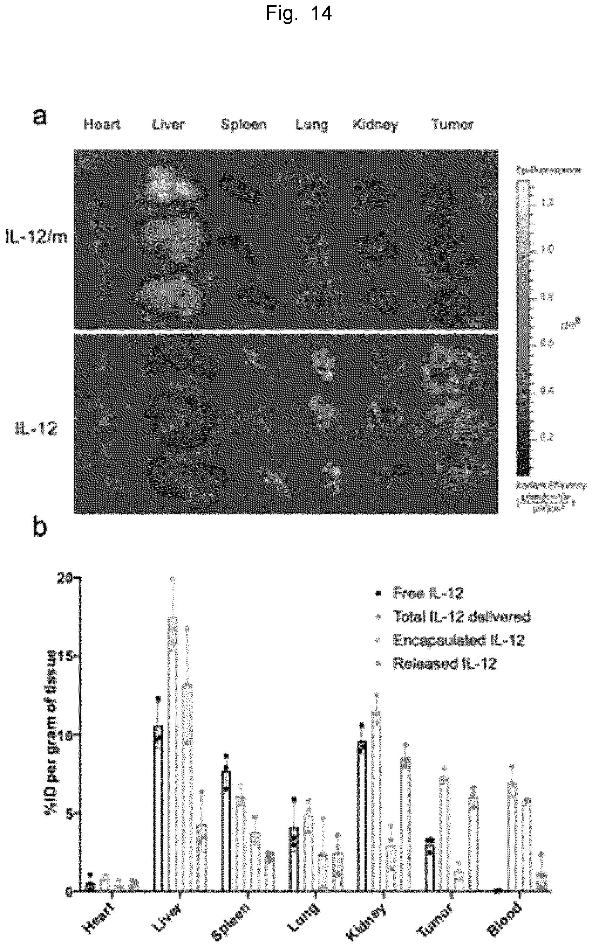

Figure 14 shows the *in vivo* distribution of IL-12/m at 24 hours after injection at high dose (10 µg IL-12 equivalence).
   a) IVIS photographs of major organs and melanoma tumor. Fluorescence intensity indicates the accumulation level of Alexa 647-IL-12. b) Quantification results obtained from ELISA assay on TNBC tumor-bearing mice. The level of encapsulated IL-12 (green column) was calculated by subtracting the value of released IL-12 (blue column) from the level of total IL-12 delivered (red column). Data were normalized to the initial dose. (n = 3)

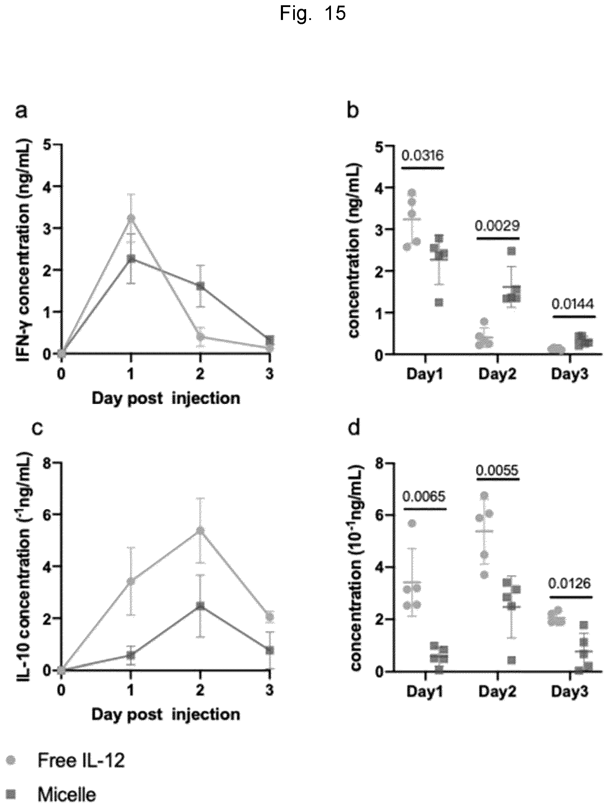

Figure 15 shows the systemic secretion of IFN-γ and IL-10 after low dose treatment.
   1 µg of IL-12 or IL-12/m equivalence was intravenously injected on Day 0. The plasma concentrations of IFN-γ and IL-10 were measured every day. The concentrations of IFN-γ (a) and IL-10 (c) were plotted against time to calculate AUC. The concentrations of IFN-γ (b) and IL-10 (d) in the individual samples were also shown.
   n = 5, p-values were analyzed by unpaired t-test.

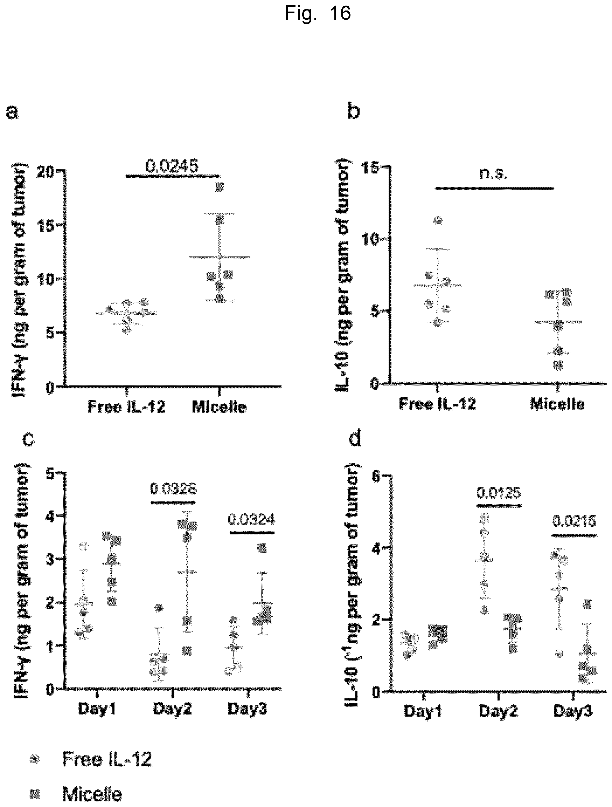

Figure 16 shows the intratumoral concentrations of IFN-γ and IL-10.
   In high dose treatment, tumor samples were taken on Day 7 for ELISA assay to detect the levels of IFN-γ (a) and IL-10 (b). In low dose treatment, tumors were taken from the treated mice on Days 1, 2 and 3 for ELISA assay to detect the levels of IFN-γ (c) and IL-10 (d).
   n = 5, p-values were analyzed by unpaired t-test.

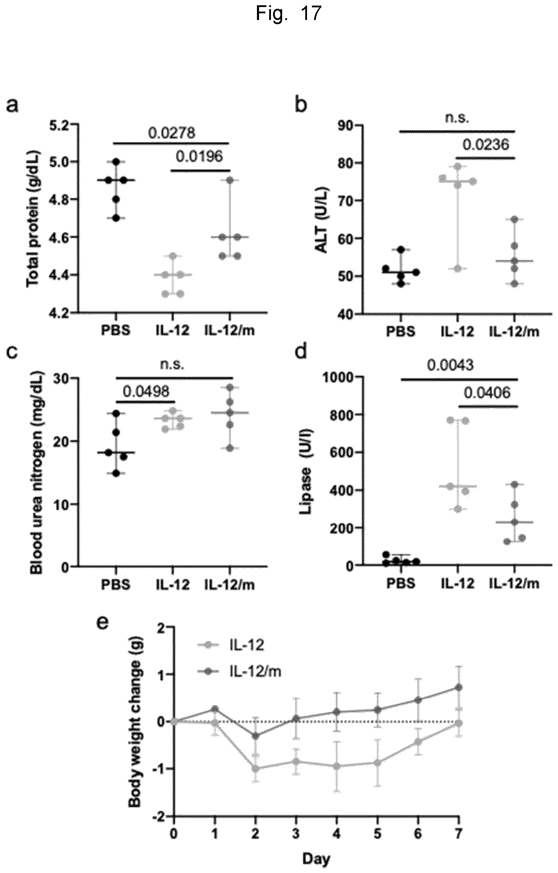

Figure 17 shows systemic toxicity indicated by blood biomarkers and body weight change.
   a) TP concentration and b) ALT concentration, which are indicative of hepatic injury, c) BUN value, which is indicative of nephropathy. d) Lipase concentration, which is indicative of pancreatic injury. e) Relative mouse body weight during the period of administration (red: IL-12-receiving group, blue: IL-12/m-receiving group). The body weight of each mouse on Day 0 was set as a zero point for each mouse.
   n = 6, p-values were analyzed by unpaired t-test.

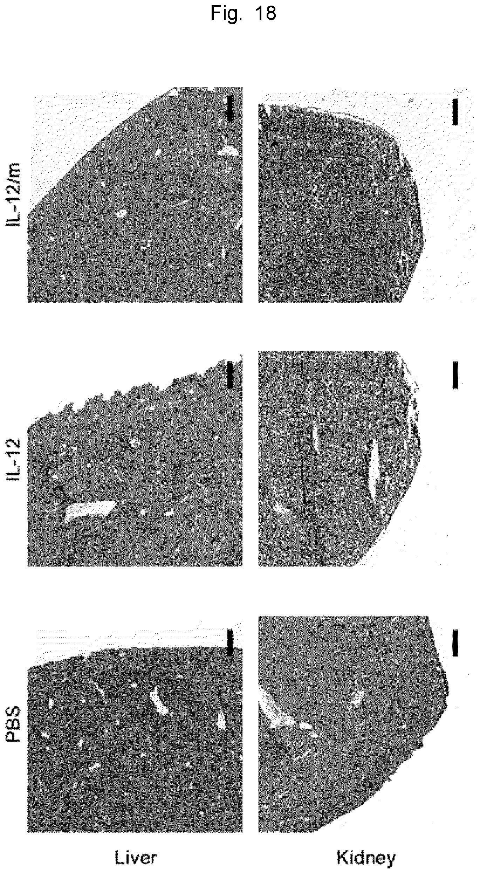

Figure 18 shows the representative results of H&E staining of the liver and kidney.
   In all the samples, no visual pathological change was observed. Scale bar = 100 µm.

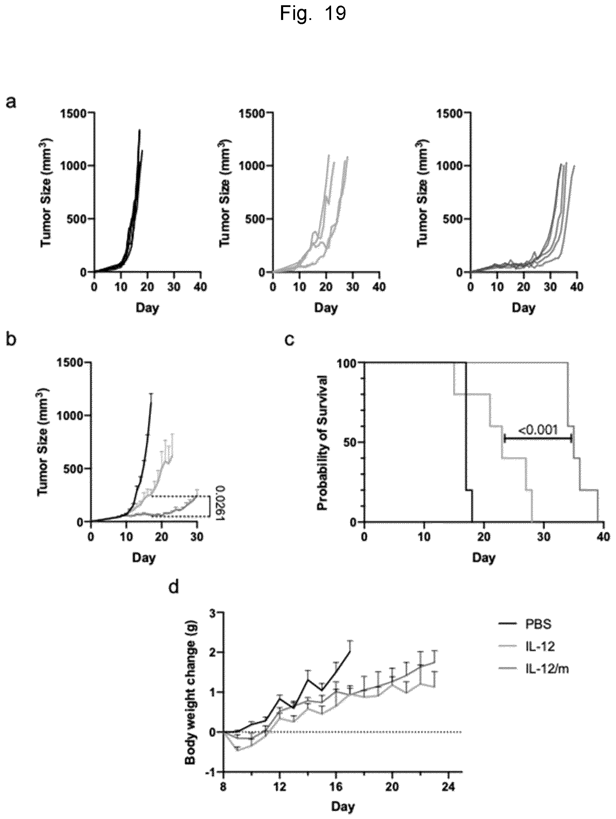

Figure 19 shows the antitumor activity of IL-12/m in a melanoma model.
   IL-12 10 µg or equivalent IL-12/m was intravenously administered to mice on Day 8. a) Individual tumor growth curves. b) Average tumor size of each treated group. c) Survival curve of each group. d) Average body weight changes in mice under treatment.
   n = 5, the tumor size was analyzed by unpaired t-test, and the survival rate was analyzed by Log-rank test.

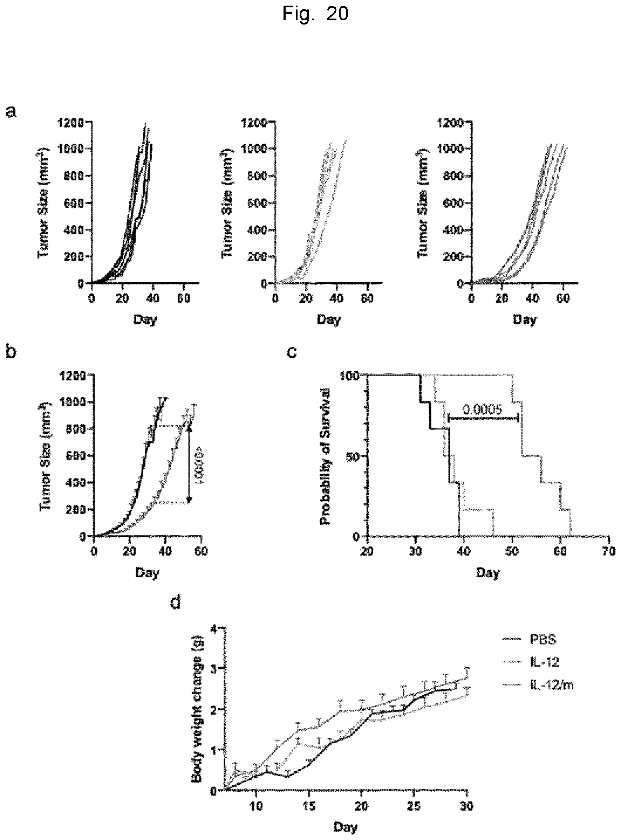

Figure 20 shows the antitumor activity of IL-12/m in a TNBC model.
   10 µg of IL-12 or equivalent IL-12/m was intravenously injected on Day 7.
      a) Individual tumor growth curves. b) Average tumor size of each treated group. c) Survival curve of each group. d) Average body weight changes in mice under treatment.
   n = 5, the tumor size was analyzed by unpaired t-test, and the survival rate was analyzed by Log-rank test.

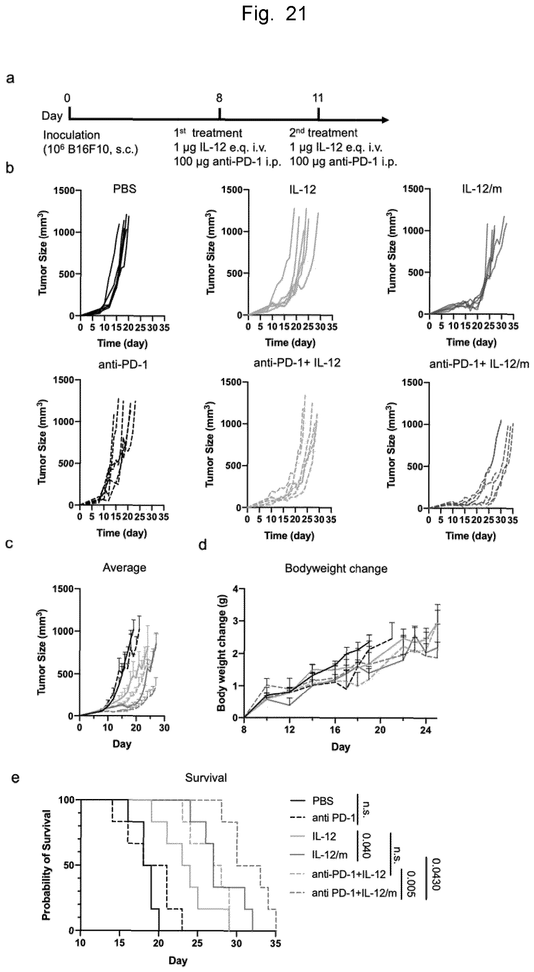

Figure 21 shows the antitumor activity of IL-12/m in a melanoma model under a low dose multi-injection treatment schedule.
   a) Scheme showing the treatment schedule in the experiment. Treatment was repeated twice. The first treatment was performed on Day 8 after tumor inoculation. For the second treatment, IL-12 1 µg or IL-12/m equivalence was intravenously administered at 3 days after the first treatment (i.e., on Day 11), and anti-PD-1 was intraperitoneally administered. b) Individual tumor growth curves. c) Average tumor size of each treated group. d) Body weight changes in mice under treatment. e) Survival curve of each group.
   n = 6, the survival rate was analyzed by Log-rank test.

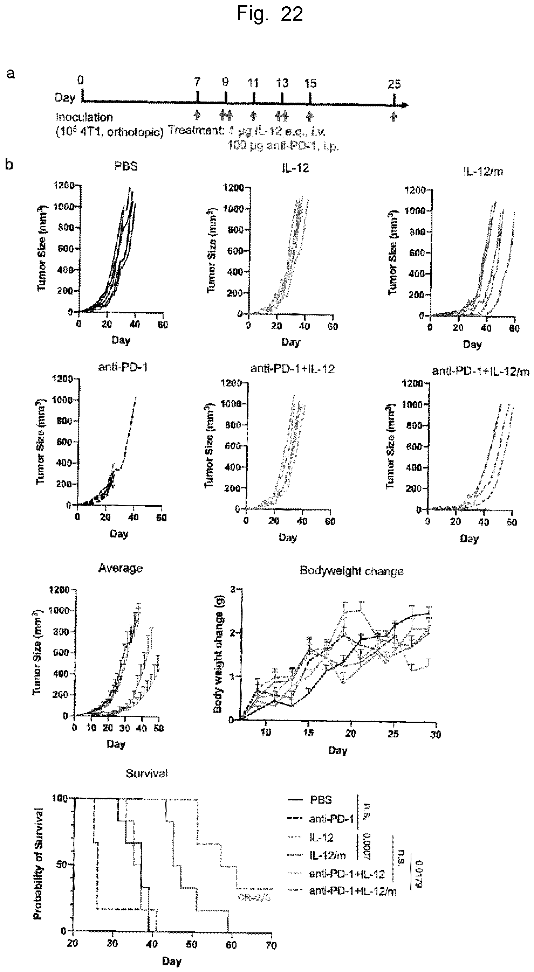

Figure 22 shows the antitumor activity of IL-12/m in a TNBC model under a low dose (1 µg) multi-injection treatment schedule.

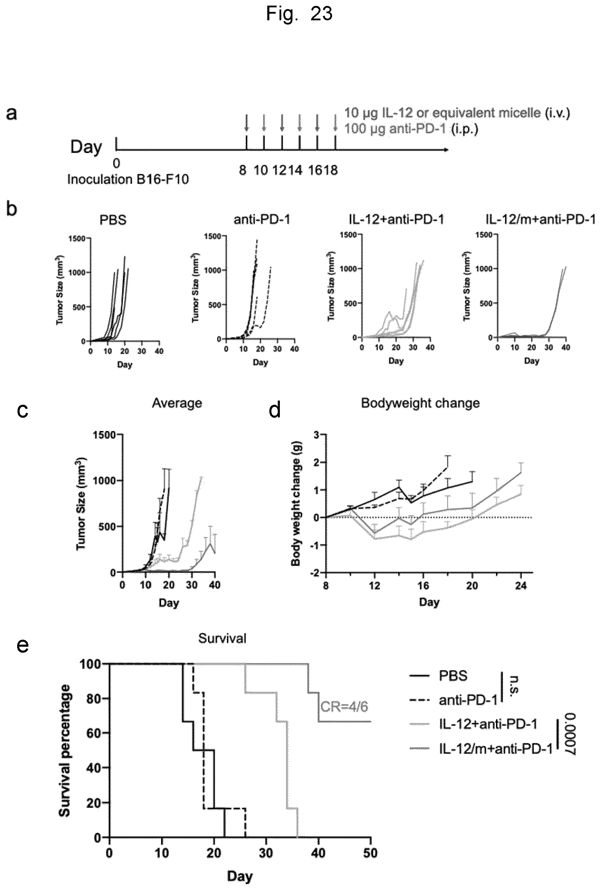

Figure 23 shows the antitumor activity of IL-12/m in a melanoma model under a high dose multi-injection treatment schedule combined with anti-PD-1.
   a) Scheme showing the treatment schedule in the experiment. IL-12/m or IL-12 treatment was repeated three times. The first treatment was performed on Day 8 after tumor inoculation. The treatment was then performed every 4 days. Anti-PD-1 was injected three times on Day 10, Day 14 and Day 18, respectively. b) Individual tumor growth curves. c) Average tumor size of each treated group. d) Body weight changes in mice under treatment. e) Survival curve of each group.
   n = 6, the survival rate was analyzed by Log-rank test.

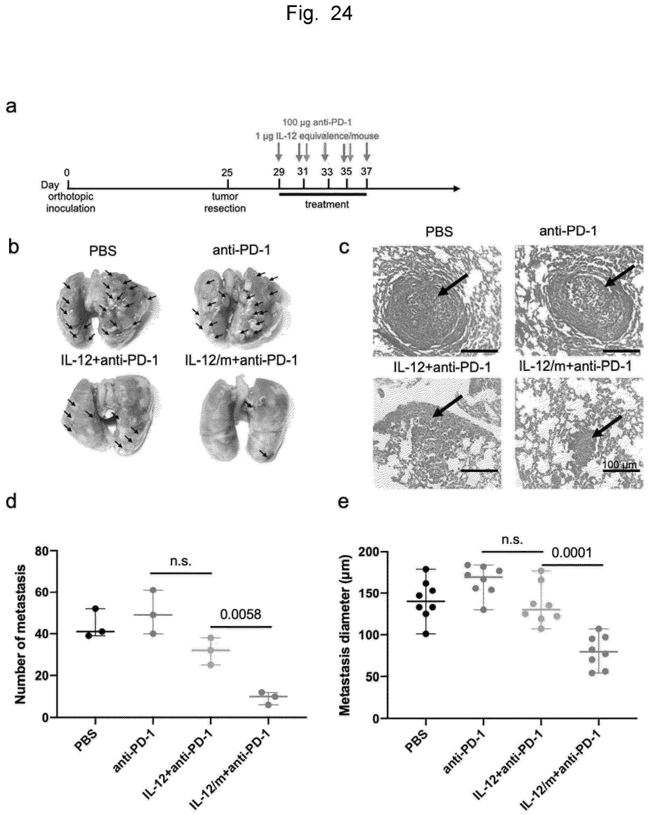

Figure 24 shows the anti-metastatic activity of IL-12 or IL-12/m upon combined use with anti-PD-1.
   a) Scheme showing the treatment schedule. IL-12 and IL-12/m were injected five times at low dose (1 µg of IL-12 equivalence per injection). The first injection was performed at 4 days after operation (i.e., on Day 29). The injection was then performed every two days. Anti-PD-1 was injected twice on Day 31 and Day 35, respectively. b) Photographs of lung analytes taken on Day 35 (i.e., at 10 days after operation). Metastatic spots were indicated with arrows, c) H&E stained sections showing the size of metastatic spots (indicated with arrows). Scale bar = 100 µm, n = 3. d) Statistical results of lung metastasis counts on Day 35, n = 8. e) Statistical results of lung metastasis size on the H&E sections.
   p-values were analyzed by unpaired t-test.

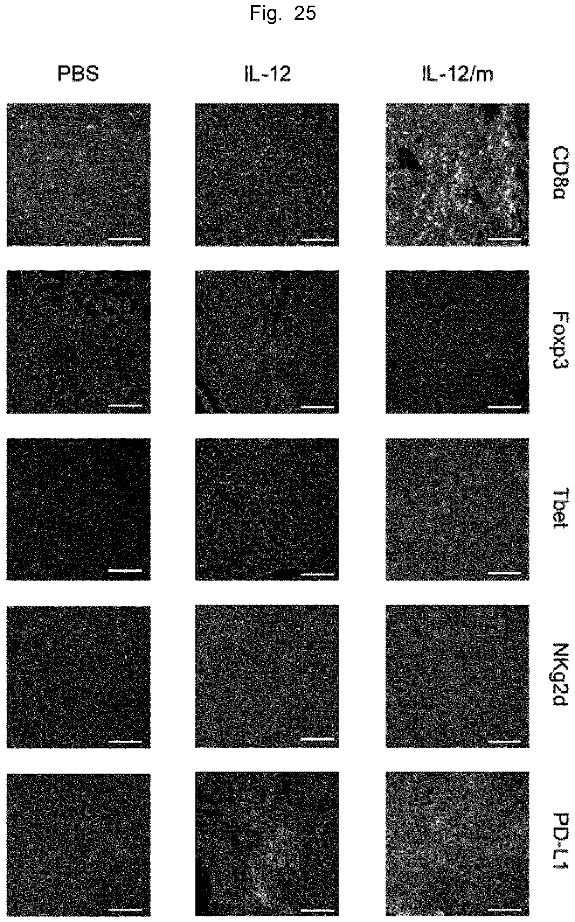

Figure 25 shows the representative IHC results of TNBC tumor sections.
   When tumors had grown to 50 mm³, mice were treated with 10 µg of IL-12 or equivalent IL-12/m, and tumor tissue samples were taken from the mice at 5 days after treatment. Scale bar = 100 µm.

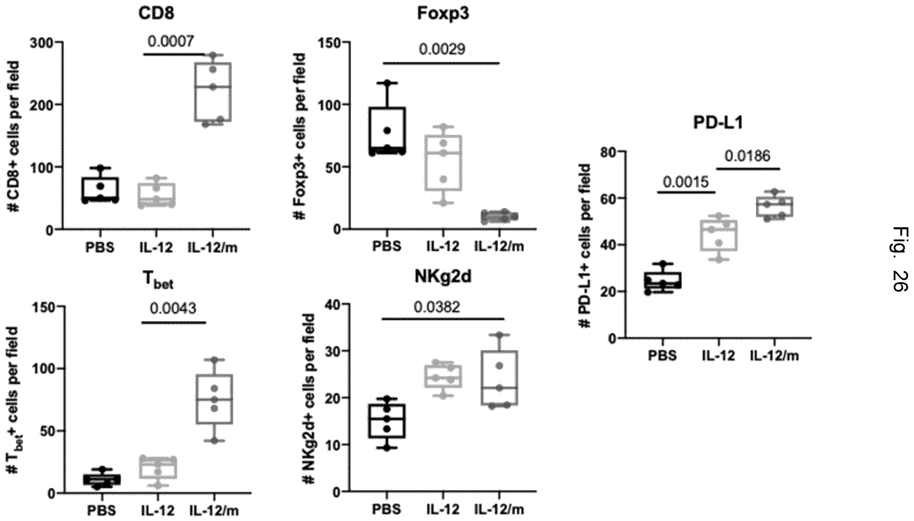

Figure 26 shows the quantitative analysis results of the IHC results shown in Figure 25.
   Five different fields were analyzed for each group.
   n = 5, p-values were analyzed by unpaired t-test.

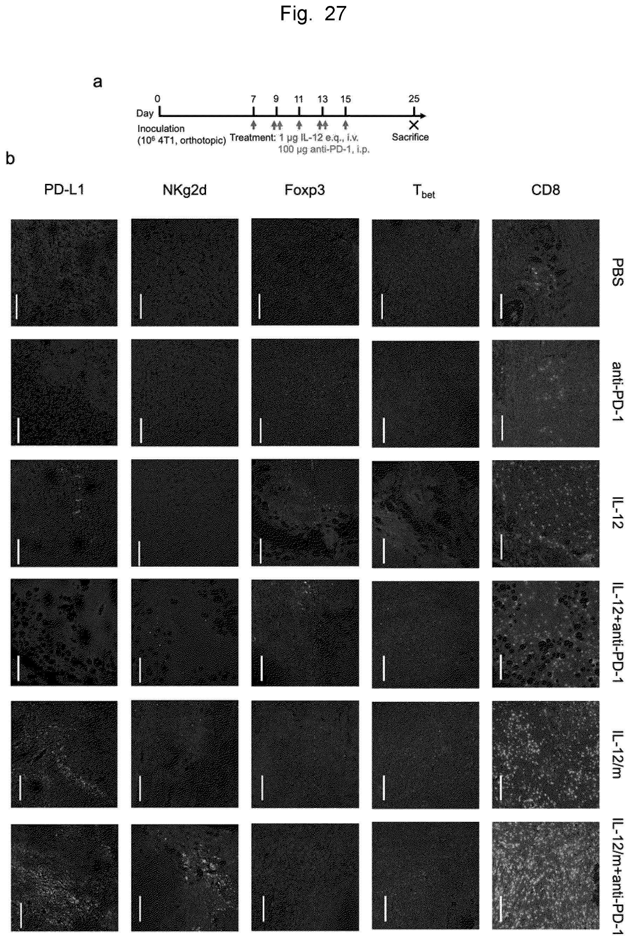

Figure 27 shows the IHC results of low dose multi-treatment in TNBC tumor.
   a) Scheme showing details of the treatment schedule. b) Representative results in corresponding groups. Scale bar = 100 µm.

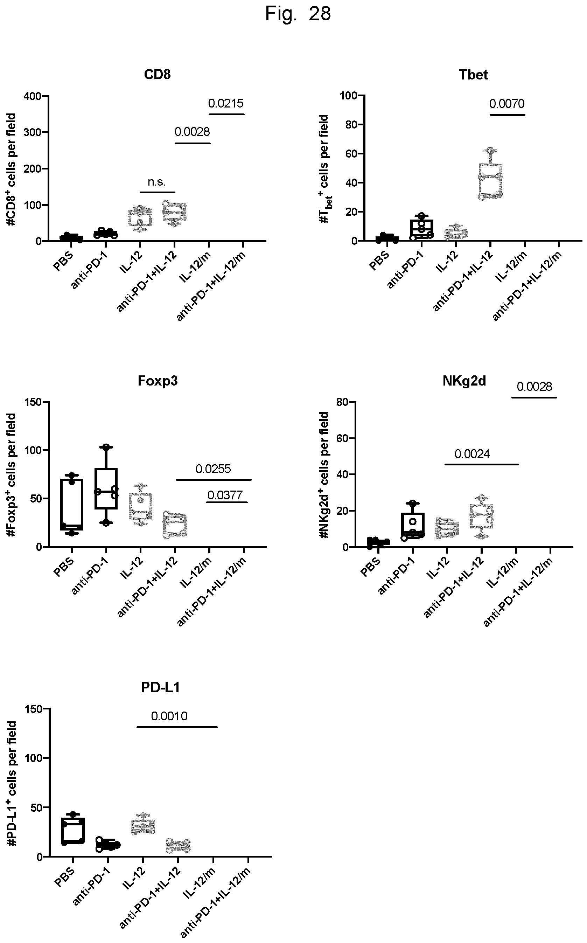

Figure 28 shows the quantitative analysis results of the IHC results shown in Figure 27.
   Five different fields were analyzed for each group.
   n = 5, p-values were analyzed by unpaired t-test.

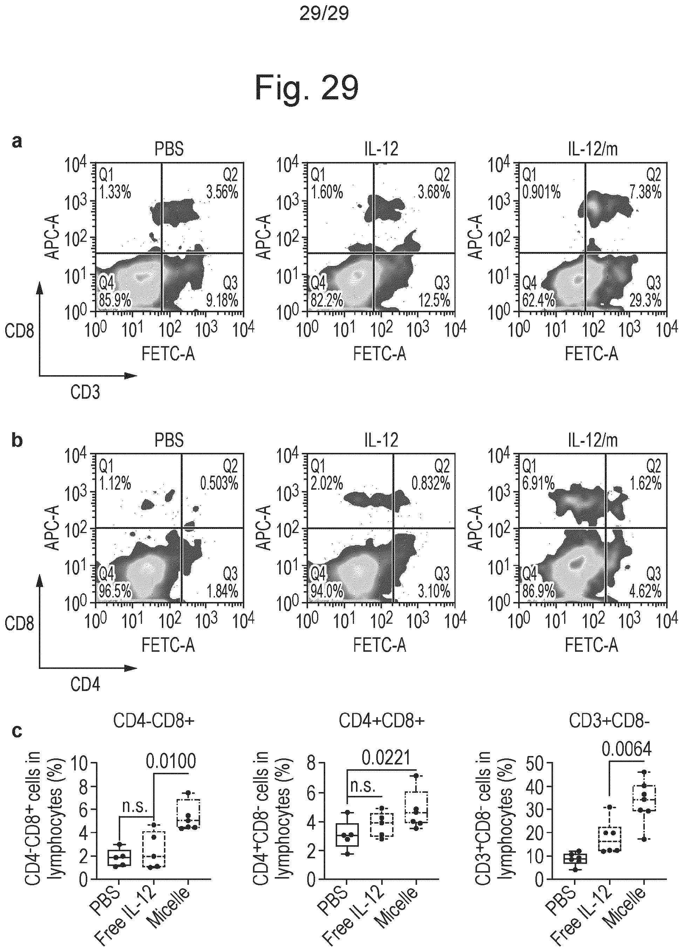

Figure 29 shows the FCM results of tumor samples after low dose multi-treatment.
   In this test, mice bearing melanoma (average size: 50 mm³) were intravenously injected twice with 1 µg of IL-12 or equivalent micelle. The second injection was performed at 2 days after the first injection. At 5 days after the second injection, tumors were taken from the mice. Single cell suspensions were prepared from the tumor samples, stained with corresponding antibodies, and then provided for detection with a flow cytometer.
      a) Representative scatter diagrams of CD3/CD8 double staining. b) Representative scatter diagrams of CD4/CD8 double staining, c) Statistical results quantified from the FCM measurements shown in (a) and (b).
   n = 6, p-values were analyzed by unpaired t-test.

### Description of Embodiments

Although therapeutic proteins are expected to be promising in the treatment of intractable diseases, their systemic administration involves various problems including instability, short half-life, and non-specific immune reactions, etc. Thus, a protein delivery approach using stimuli-responsive nanocarriers may be an effective strategy to enhance protein activity in target tissues in a tissue selective manner. In the present invention to load a cytokine as a protein, there have been developed polymeric micelles having the ability to form a polyion complex between cytokine and block copolymer and thereby encapsulate the cytokine through covalent bonding cleavable under given pH conditions, with the aim of releasing the cytokine in a pH-dependent manner.

A carboxydimethylmaleic anhydride (CDM)-amide bond is stable at physiological pH (pH 7.4), but is cleaved at pH 6.5, i.e., at pathophysiological pH in tumors and inflammatory tissues. For this reason, CDM was selected as a pH-responsive functional group. In the present invention, a poly(ethylene glycol)-poly(L-lysine) block copolymer with 45% CDM addition was used, whereby interleukin 12 (IL-12) was encapsulated with an efficiency of 50% or higher. IL-12-encapsulating micelles (IL-12/m) were used as a model to confirm micelle stability under physiological conditions, as well as micelle breakdown and functional IL-12 release at pH 6.5. Further, IL-12/m were found to have an improved blood half-life when compared to IL-12 alone and covalent bond-free micelles associated only by electrostatic interaction. Thus, the above model indicated the usefulness of the system for *in vivo* delivery of cytokines.

The CDM-amide bond is unstable at pH 6.5^{[17-19]} and thereby allows release of the conjugated amino compound at pathological pH, so that CDM was selected as a pH-responsive site in the present invention. Thus, the resulting cytokine-encapsulating micelles each form a stable crosslinked core at physiological pH, but are degraded at pH 6.5 into a free block copolymer and an active cytokine. In the present invention, these micelles were evaluated for their ability to encapsulate cytokines, particularly IL-12. Further, the inventors of the present invention used micelles encapsulating IL-12 as a model to evaluate their *in vitro* stability and IL-12 release at different pHs, as well as their *in vivo* blood retention after systemic administration.

### 1. Polymeric complex of the present invention

The polymeric complex of the present invention is a protein-encapsulating polymeric micellar complex (polyion complex: PIC), which comprises a particular type of cationic polymer (e.g., block copolymer, graft copolymer) and a protein (the details of this protein will be described later).

### (1) Cationic polymer

A particular type of cationic polymer, which is a member constituting the PIC of the present invention, is a cationic polymer at least partially having a polycation moiety. Such a cationic polymer may be, for example, a block copolymer or graft polymer having a polyethylene glycol (PEG) moiety and a polycation moiety, without being limited thereto. Depending on the intended use of the PIC of the present invention, a preferred embodiment may be selected as appropriate.

The above PEG and polycation have no limitation on their structure (e.g., their degree of polymerization), and those of any structure may be selected. Above all, preferred as a polycation is a polypeptide having cationic groups in its side chains. As used herein, the term "cationic group" is intended to mean not only a group which is already cationic by being coordinated with hydrogen ions, but also a group which will be cationic when coordinated with hydrogen ions. Such cationic groups include all of the known ones. A polypeptide having cationic groups in its side chains is intended to include those composed of known amino acids having a basic side chain (e.g., lysine, arginine, histidine) linked via peptide bonds, as well as those composed of various amino acids linked via peptide bonds, whose side chain (e.g., the side chain of aspartic acid or glutamic acid) is substituted to have a cationic group.

More specifically, the above particular type of cationic polymer may preferably be exemplified by a block copolymer represented by the following general formula (1).

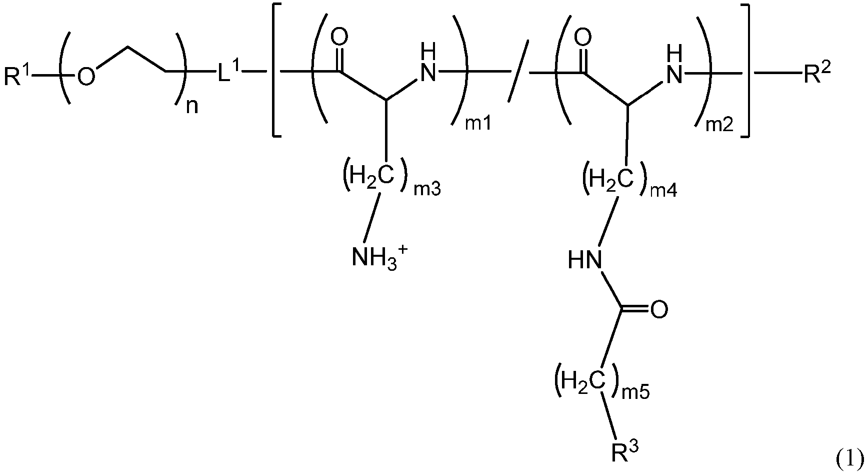

In the structural formula shown in general formula (1), the block moiety whose number of repeating units (degree of polymerization) is n corresponds to the PEG moiety, while the block moiety composed collectively of submoieties whose number of repeating units is m1 and m2, respectively (i.e., the moiety shown in brackets [ ] in general formula (1)) corresponds to the polycation moiety. Moreover, the symbol "/" appearing in the structural formula of the polycation moiety is intended to mean that the respective monomer units shown on the left and right sides of this symbol may be in any sequence. For example, when a block moiety composed of monomer units A and B is represented by [-(A)a-/-(B)b-], the symbol "/" means that a units of A and b units of B, i.e., (a + b) units in total of the respective monomer units may be linked at random in any sequence (provided that all the monomer units A and B are linked in a linear fashion).

In general formula (1), R¹ and R² each independently represent a hydrogen atom, or an optionally substituted linear or branched alkyl group containing 1 to 12 carbon atoms, or a functional group such as an azide, an amine, maleimide, a ligand or a labeling agent.

Examples of the above linear or branched alkyl group containing 1 to 12 carbon atoms include a methyl group, an ethyl group, a n-propyl group, an isopropyl group, a n-butyl group, a sec-butyl group, a tert-butyl group, a n-pentyl group, a n-hexyl group, a decyl group and an undecyl group, etc. Moreover, examples of substituents on the above alkyl group include an acetal-protected formyl group, a cyano group, a formyl group, a carboxyl group, an amino group, an alkoxycarbonyl group containing 1 to 6 carbon atoms, an acylamido group containing 2 to 7 carbon atoms, a siloxy group, a silylamino group, and a trialkylsiloxy group (each alkylsiloxy group independently contains 1 to 6 carbon atoms), etc.

A ligand molecule refers to a compound used with the aim of targeting a certain biomolecule, and examples include an antibody, an aptamer, a protein, an amino acid, a low molecular compound, a monomer of a biological macromolecule and so on. Examples of a labeling agent include, but are not limited to, fluorescent labeling agents such as a rare earth fluorescent labeling agent, coumarin, dimethylaminosulfonyl benzoxadiazole (DBD), dansyl, nitrobenzoxadiazole (NBD), pyrene, fluorescein, a fluorescent protein and so on.

When the above substituent is an acetal-protected formyl group, this substituent can be converted into another substituent, i.e., a formyl group (or an aldehyde group; - CHO) upon hydrolysis under acidic mild conditions. Moreover, when the above substituent (particularly on R¹) is a formyl group or is a carboxyl group or an amino group, for example, an antibody or a fragment thereof or other functional or targeting proteins may be linked via these groups.

In general formula (1), R³ represents a compound represented by the following general formula (I).

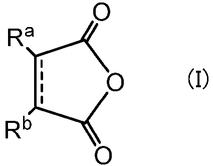

In the above formula (I), R^{a} and R^{b} each independently represent a hydrogen atom, or an optionally substituted alkyl group, an alkenyl group, a cycloalkyl group, an aryl group, an aralkyl group, an acyl group, a heterocyclic group, a heterocyclic alkyl group, a hydroxy group, an alkoxy group or an aryloxy group. Alternatively, R^{a} and R^{b} may be joined to form an aromatic ring or a cycloalkyl ring together with the carbon atoms to which they are attached respectively. Moreover, in formula (I), the bond between the carbon atoms to which R^{a} and R^{b} are attached respectively may be a single bond or a double bond, i.e., is not limited in any way. In formula (I), to express these two bonding modes collectively, the bond between these carbon atoms is represented by a combination of one solid line and one broken line.

L¹ represents NH, CO, a group represented by the following general formula (11):

-(CH₂)ₚ₁-NH- (11)

(wherein p1 represents an integer of 1 to 6), or
a group represented by the following general formula (12):

   -L^{2a}-(CH₂)_{q1}-L^{3a}- (12)
(wherein L^{2a} represents OCO, OCONH, NHCO, NHCOO, NHCONH, CONH or COO, L^{3a} represents NH or CO, and q1 represents an integer of 1 to 6).

In the above formula (1), m1 and m2 each independently represent an integer of 0 to 500 (provided that the sum of m1 and m2 represents an integer of 10 to 500), and m3, m4 and m5 each independently represent an integer of 1 to 5. In the above formula (1), n represents the number of repeating units (degree of polymerization) in the PEG moiety, and more specifically represents an integer of 1 to 500 (preferably 100 to 400, more preferably 200 to 300).

The molecular weight (Mn) of the cationic polymer represented by general formula (1) is not limited in any way, but it is preferably 23,000 to 45,000, and more preferably 28,000 to 34,000. With regard to the individual block moieties, the PEG moiety has a molecular weight (Mw) of preferably 8,000 to 15,000, and more preferably 10,000 to 12,000, while the polycation moiety as a whole has a molecular weight (Mn) of preferably 15,000 to 30,000, and more preferably 18,000 to 22,000.

The cationic polymer represented by general formula (1) may be prepared in any manner. For example, a segment comprising R¹ and the block moiety of PEG chain (PEG segment) is synthesized in advance, and given monomers are sequentially polymerized to one end (opposite to R¹) of this PEG segment, optionally followed by substituting or converting each side chain to contain a cationic group, or alternatively, the above PEG segment and a block moiety containing cationic groups in its side chains are synthesized in advance, which are then liked to each other. Procedures and conditions for each reaction in these preparation processes may be selected or determined as appropriate in consideration of standard processes.

In one embodiment of the present invention, the compound represented by formula (I) is at least one of compounds represented by the following formulae (Ia) to (Ig).

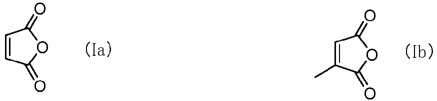

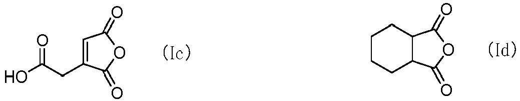

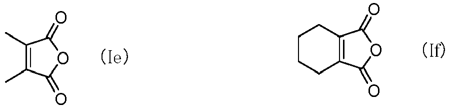

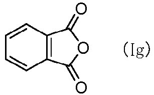

In a preferred embodiment of the present invention, the compound represented by formula (I) is a compound represented by the following formula (Ia) or (Ib).

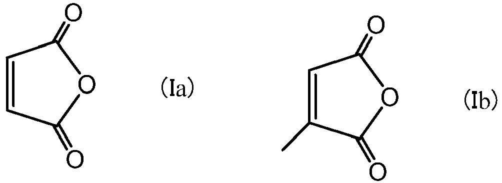

In formula (I), possible substituents may be saturated or unsaturated non-cyclic or cyclic hydrocarbon groups. In the case of non-cyclic hydrocarbon groups, they may be either linear or branched. Examples of such hydrocarbon groups include a C₁-C₂₀ alkyl group, a C₂-C₂₀ alkenyl group, a C₄-C₂₀ cycloalkyl group, a C₆-C₁₈ aryl group, a C₆-C₂₀ aralkyl group, a C₁-C₂₀ alkoxy group, and a C₆-C₁₈ aryloxy group.

The compound represented by formula (I) is used as a charge regulator. The compound represented by formula (I) acts to convert the charge of a basic or neutral protein as a whole into that of an acidic protein. In other words, the charge regulator of the present invention is deemed to cause overall charge conversion by controlling the amount of charge such that a protein whose overall charge is positive (+) or in neutral state is converted into a protein whose overall charge is negative (-). More specifically, the above overall charge conversion is accomplished as follows: the above compound represented by formula (I) or a derivative thereof is bonded to an amino group (i.e., a positively charged group) contained in a protein, whereby the protein is negatively charged as a whole. For this purpose, this bonding is accomplished, for example, as follows: the above compound represented by formula (I) is bonded (covalently bonded) to an amino group in a protein to form a structure as represented by the following formula (I').

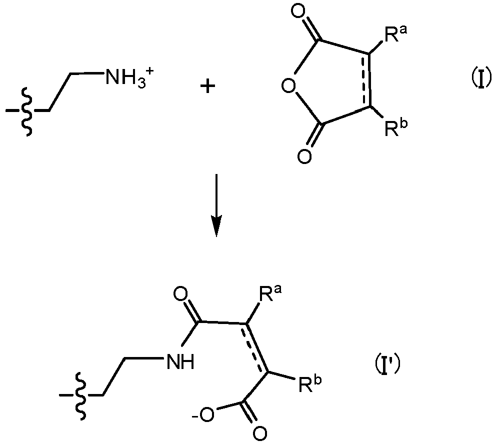

As to the above bonding, for example, when the above compound represented by formula (I) is a compound represented by formula (Ib) or (Ic) shown above, the above structure represented by formula (I') formed after the bonding is as shown below.

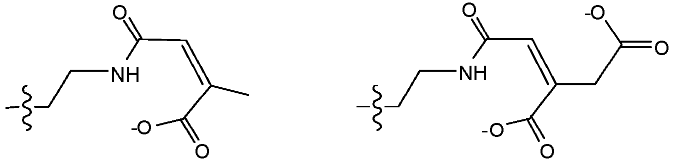

In a further embodiment of the present invention, the block copolymer represented by formula 1 is represented by the following formula 2.

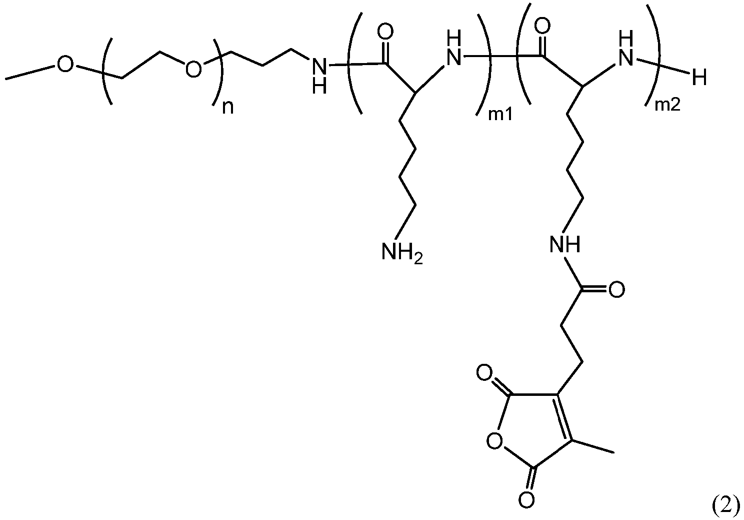

### (2) Cytokine

In the PIC of the present invention, a protein serving as a member constituting the core region may be a protein whose charge has been converted as a whole by the above compound represented by formula (I) (i.e., a charge-conversional protein), and more specifically may be a protein whose overall charge has been converted from the overall charge of a basic or neutral protein (which is positive or in neutral state) into a negative charge, as in the case of the overall charge of an acidic protein. Such a protein whose overall charge has been converted into a negative charge can be regarded as an anionic substance (polyanion) when the protein is taken as a whole. Thus, upon electrostatic interaction with the polycation moiety in the above cationic polymer, such a charge-conversional protein can easily form a micellar complex which is inherently difficult to form with a basic or neutral protein.

In the present invention, a cytokine is used as a protein.

Cytokines are low molecular proteins secreted from cells and are involved in cell-cell interactions to thereby affect surrounding cells. As a result, cell-mediated immunological effects and antitumor effects are exerted.

Cytokines include interleukins (IL) (e.g., IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21), hematopoietic factors (CSF, EPO, TPO), interferons (IFN), tumor necrosis factors (TNF), growth factors (EGF, FGF, PDGF) and son on. Among them, IL-12 can be used in the present invention.

Moreover, the cytokine to be used in the present invention is not limited to those consisting of full-length amino acid sequences, and also encompasses their partial fragments and peptides, etc. Moreover, the cytokine to be used in the present invention is not limited to those composed of natural amino acids, and also encompasses modified cytokines comprising at least some unnatural amino acids as constituent members. Furthermore, the cytokine to be used in the present invention also encompasses those modified as appropriate to have various labeling substances or the like, if necessary.

### (3) Polyion complex (PIC)

The PIC of the present invention can be regarded as a core-shell type micellar complex in such a state where the cytokine and a part (polycation moiety) of the above cationic polymer form a core region through their electrostatic interaction, and other parts (including the PEG moiety) in the cationic polymer form a shell region around the core region.

The PIC of the present invention may be readily prepared by mixing the cytokine (e.g., IL-12) and the cationic polymer in any buffer (e.g., Tris buffer). The mixing ratio between the cationic polymer and the cytokine is not limited in any way. However, in the present invention, for example, the ratio between the total number (N) of cationic groups (e.g., amino groups) in the block copolymer and the total number (C) of carboxyl groups in the cytokine (N/C ratio) may be set to 0.1 to 200, particularly 0.5 to 100, and more particularly 1 to 50. If the N/C ratio is within the above range, it is preferred in that free molecules of the cationic polymer can be reduced. It should be noted that the above cationic groups (N) are intended to mean groups capable of forming ionic bonds through electrostatic interaction with carboxyl groups in the cytokine to be encapsulated within the micelle.

The PIC of the present invention is of any size. For example, its particle size is preferably 5 to 200 nm, and more preferably 10 to 100 nm, as measured by dynamic light scattering (DLS).

Upon introduction into cells, the PIC of the present invention will release the cytokine encapsulated therein. In this case, the above compound represented by formula (I) is dissociated (cleaved) from the cytokine in response to a change in the pH environment within the cytoplasm (which is changed to a weakly acidic environment (e.g., around pH 5.5)). As a result, the charge (overall charge) of the cytokine as a whole returns to the original charge (overall charge) inherent to the cytokine, so that the cytokine can be present within the recipient cells in a state where its structure and activity, etc. are regenerated.

### 2. Cytokine delivery device

The present invention provides a cytokine delivery device comprising the above polyion complex (PIC). The cytokine delivery device of the present invention can be used as a means to efficiently introduce a cytokine encapsulated within the core region of PIC into any site in target cells selected from a cell surface site, an intracellular site and an extracellular site, with the aid of changes in the oxidation-reduction environment between inside and outside of the cells.

More specifically, a solution containing PIC encapsulating a cytokine is administered to an animal subject and taken up into target cells in the body. Then, once the PIC taken up into the cells has reached endosomes, the compound represented by formula (I) will be liberated from the cytokine to cause a change in the charge balance within the PIC, whereby the PIC will be broken down. Once the PIC has been broken down, the cytokine will be released from the PIC, and the polymer dissociated at the same time from the PIC will damage the endosomal membrane. As a result, the endosomes are destructed to achieve delivery of the released cytokine into the cytoplasm.

In the case of micelles encapsulating a cytokine, the cytokine is released outside of cells and binds to its receptor on the cell surface, so that delivery can be targeted to cell surface sites.

The cytokine delivery device of the present invention may be applied to various mammals including, but not limited to, humans, mice, rats, rabbits, pigs, dogs and cats. For administration to an animal subject, parenteral modes such as intravenous drip infusion are usually selected, and conditions (e.g., dosage, administration frequency and administration period) may be determined as appropriate for the type and condition of the animal subject.

### 3. Pharmaceutical composition

The complex and the cytokine delivery device of the present invention can be used in therapies intended to introduce a cytokine into cells responsible for various diseases (e.g., antitumor therapy or the like in the case of the complex encapsulating IL-12). Thus, the present invention can also provide a pharmaceutical composition (e.g., a pharmaceutical composition for use in antitumor therapy) comprising the above PIC, and a method for treating various diseases (e.g., tumor) using the above PIC. It should be noted that the administration mode and conditions are the same as those described above.

In the pharmaceutical composition of the present invention, there is no particularly limitation on the type of tumor to be applied. Examples include brain tumor (pituitary adenoma, glioma), head and neck cancer, cervical cancer, maxillary cancer, oral cancer, salivary gland cancer, sublingual gland cancer, parotid gland cancer, nasal cancer, paranasal cancer, laryngeal cancer, esophageal cancer, lung cancer, breast cancer, pancreatic cancer, gastric cancer, biliary tract cancer (bile duct cancer, gallbladder cancer), intestinal or duodenal cancer, colorectal cancer, bladder cancer, renal cancer, liver cancer, prostate cancer, uterine cancer (uterine cervical cancer, uterine body cancer), ovarian cancer, thyroid cancer, pharyngeal cancer, sarcoma (e.g., osteosarcoma, chondrosarcoma, Kaposi's sarcoma, myosarcoma, angiosarcoma, fibrosarcoma), malignant lymphoma (Hodgkin's lymphoma, non-Hodgkin's lymphoma), leukemia (including, e.g., chronic myeloid leukemia (CML), acute myeloid leukemia (AML), chronic lymphocytic leukemia (CLL) and acute lymphocytic leukemia (ALL), lymphoma, multiple myeloma (MM), myelodysplastic syndromes), skin cancer, melanoma and so on.

Moreover, for example when IL-10, IL-4, IL-10, IL-6, IL-11, IL-13 IL-1R, IL-18R, TNF-R1, TNF-R2, TGF-β or CXCL12 is used as a cytokine in the present invention, inflammatory diseases and immune system diseases can be targeted.

Examples of inflammatory diseases include rheumatism, psoriasis, multiple sclerosis, Crohn's disease, myocarditis, type I diabetes, systemic lupus erythematosus, inflammatory bowel disease, ulcerative colitis, laryngopharyngitis, bladder cystitis, hepatitis, pneumonia, pancreatitis, enteritis, multiple myositis, dermatomyositis, scleroderma, Sjogren's syndrome, Crohn's disease, multiple sclerosis, myasthenia gravis, Guillain-Barre syndrome, rapidly progressive glomerulonephritis, autoimmune hemolytic anemia, ideopathic thrombocytopenia purpura (immune thrombocytopenia), Basedow's disease, pemphigus and so on.

The above pharmaceutical composition may be prepared in a standard manner by using appropriately selected excipients, fillers, extenders, binders, wetting agents, disintegrants, lubricants, surfactants, dispersants, buffering agents, preservatives, solubilizers, antiseptics, correctives, soothing agents, stabilizers and isotonizing agents, etc., which are commonly used for drug preparation.

The pharmaceutical composition of the present invention may be in the dosage form of injections, which can be administered not only systemically through usual intravenous, intraarterial or other routes, but also topically through intramuscular, intraarticular, subcutaneous, intracutaneous or other routes. In this case, the pharmaceutical composition is usually provided in the form of unit dose ampules or multidose containers, or may be in the form of powders which are reconstituted with an appropriate carrier, e.g., sterilized water before use. Moreover, these dosage forms may be configured to contain additives commonly used in formulations. The dosage of the pharmaceutical composition will vary depending on the purpose of treatment, the form of micelles, the age of a subject to be administered, the route of administration and/or the frequency of administration, and may be changed over a wide range. The amount of a cytokine contained in the pharmaceutical composition of the present invention may be determined as appropriate by those skilled in the art. For example, the effective amount of the pharmaceutical composition of the present invention and the effective amount administered as a combination with an appropriate diluent and a pharmacologically acceptable carrier may be 1 µg to 1000 µg per kg body weight per dose, given at intervals of 1 day to 6 weeks.

### 4. Cytokine delivery kit

The cytokine delivery kit of the present invention is characterized by comprising the above block copolymer. This kit can be preferably used, for example, in immunotherapy, antitumor therapy, etc.

In the kit of the present invention, the cationic polymer may be stored in any state, and a solution or powder state may be selected in consideration of its stability (storage quality) and easiness of use, etc. The kit of the present invention may further comprise other components, in addition to the above block copolymer. Examples of other components include various buffers, various proteins to be introduced into cells (charge-conversional proteins), dissolution buffers, and instructions for use (instruction manual), etc. The kit of the present invention is used to prepare a polyion complex (PIC) whose core region is formed from a cytokine to be introduced into target cells, and the PIC thus prepared can be effectively used as a device for cytokine delivery (e.g., a device for IL-12 delivery) into target cells.

### Examples

The present invention will be further described in more detail by way of the following illustrative examples, which are not intended to limit the scope of the present invention.

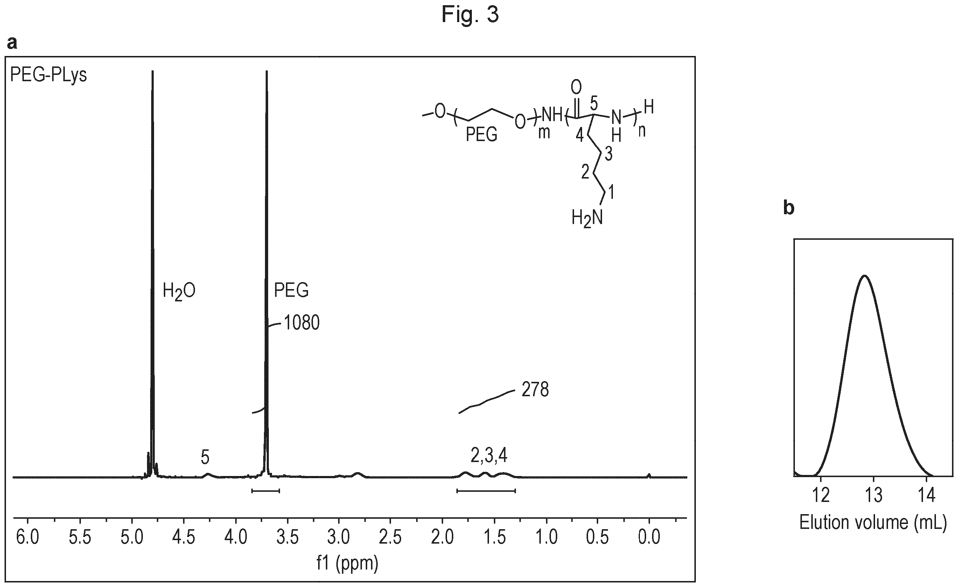

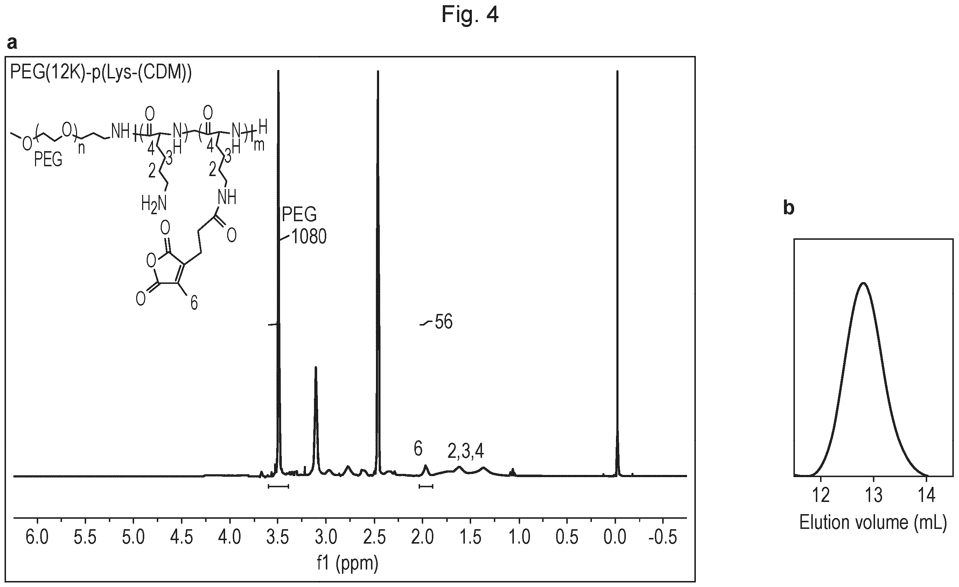

### 1. Synthesis of PEG-p(Lys-CDM) (Figures 1 to 4)

Poly(ethylene glycol)-poly(L-lysine) (PEG-p(Lys)) was synthesized by ring-opening polymerization. Thiourea (1500 mg) was dissolved in 20 mL of dehydrated DMF. MeO-PEG-NH₂ (Mw = 12 k, 500 mg) was dissolved in 5 mL of the prepared thiourea solution, and Lys(TFA)-NCA (500 mg) was dissolved in another 5 mL of the thiourea solution. Subsequently, the Lys(TFA)-NCA solution was added to the MeO-PEG-NH₂ solution under an argon atmosphere. The mixture was reacted at 35°C for 3 days, and then precipitated against cold ether. The resulting polymer PEG-pLys(TFA) was characterized by GPC (TOSOH HLC-8220 system, TOSOH, Japan; column: TSK gel G4000HHR; mobile phase: DMF; flow rate: 0.75 mL/minute; detector: UV 220 nm) and ¹H-NMR (400 MHz, JASCO, Japan) in d⁶-DMSO.

For deprotection of the TFA group, PEG-p(Lys)(TFA) was dissolved in a 1 M NaOH-containing methanol solution and reacted at 35°C for 12 hours. The mixture was dialyzed for 2 days against DI water in a 6 to 8 k MWCO membrane, and then lyophilized to obtain PEG-p(Lys). PEG-p(Lys) was characterized by GPC (JASCXO LC-EXTREMA, JASCO, Japan; column: Superdex 200-10/300 GL; mobile phase: D-PBS; flow rate: 0.75 mL/minute; detector: UV 220 nm) and ¹H-NMR in D₂O. Subsequently, CDM (100 mg) was dissolved in 8 mL of anhydrous CH₂Cl₂, and 2 mL of oxalyl chloride was added thereto, followed by reaction overnight at room temperature. For removal of residual oxalyl chloride and CH₂Cl₂, the mixture was dried under reduced pressure to obtain CDM-Cl as an oil. Then, CDM-Cl was dissolved again in 2 ml of anhydrous CH₂Cl₂, and 8 mL of CH₂Cl₂ containing PEG-p(Lys) (100 mg) was added thereto, followed by reaction overnight at room temperature.

The synthesis scheme of PEG-p(Lys-CDM) is shown in Figure 1.

The final product PEG-p(Lys-CDM) was collected by precipitating the mixture against cold ether. PEG-p(Lys-CDM) was characterized by GPC (JASCXO LC-EXTREMA, JASCO, Japan; column: Superdex 200-10/300 GL; mobile phase: pH 3.3 acetate buffered saline containing 10 mM acetate and 500 mM NaCl; flow rate: 0.75 mL/minute; detector: UV 220 nm) and ¹H-NMR in d⁶-DMSO.

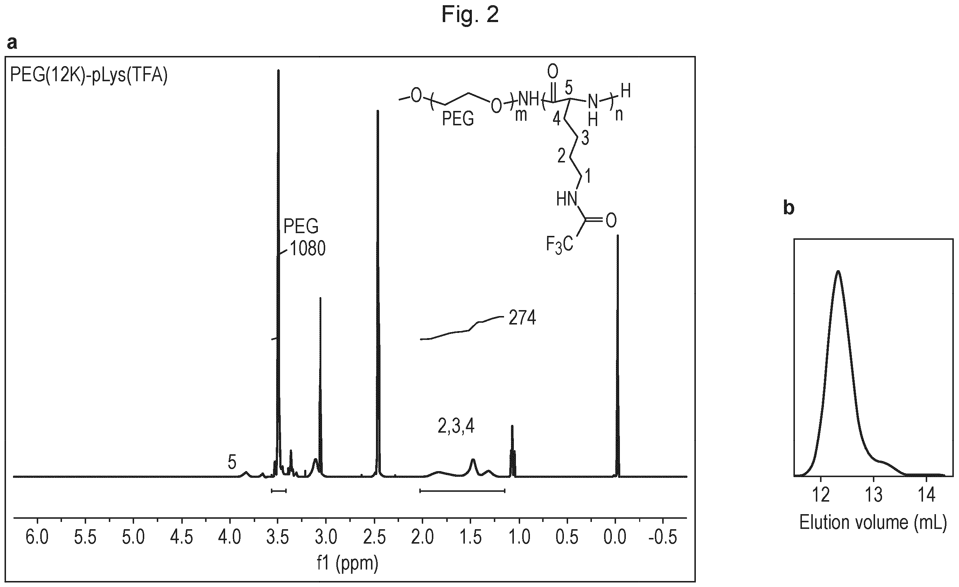

The results obtained are shown in Figures 2 to 4. As can be seen from Figures 2 to 4, PEG-p(Lys-CDM) is a polymer having a narrow molecular weight distribution and having CDM introduced into the side chain structure of p(Lys).

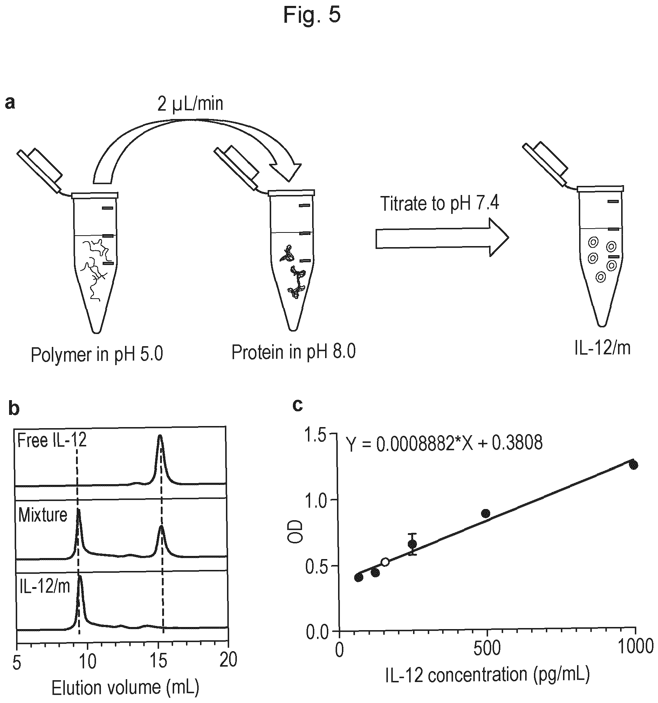

### 2. Preparation of IL-12-encapsulating micelle (IL-12/m) (Figures 5 and 6)

IL-12/m was prepared by pH-controlled titration. To avoid the formation of empty micelles, 10 mg/mL PEG-p(Lys-CDM) was prepared in phosphate buffer of pH 5.0 (20 mM). 40 µg/mL IL-12 was prepared in phosphate buffer of pH 8.0 (20 mM). To prepare IL-12/m, the mass ratio between PEG-p(Lys-CDM) and IL-12 was set to 250:1. The PEG-p(Lys-CDM) solution was added at a precisely controlled rate (2 µL/minute) to the IL-12 solution. Then, the mixture was titrated to pH 7.4 by adding PBS buffer of pH 8.0, and subsequently incubated overnight at 4°C with continuous shaking.

To determine the efficiency of encapsulation, Alexa-647-labeled IL-12 was used, and the titrated mixture was loaded onto HPLC (JASCXO LC-EXTREMA, JASCO, Japan) (column: Superdex 200-10/300 GL, GE Healthcare, USA; mobile phase: pH 7.4 PBS; flow rate: 0.75 mL/minute; detector: fluorescence 650/665 nm). The efficiency of encapsulation was determined by the area ratio between peaks derived from IL-12/m and free IL-12. In a biological experiment using non-labeled IL-12, the efficiency of encapsulation was determined by ELISA assay for detection of non-encapsulated IL-12 in the mixture.

Then, this mixture was purified by ultrafiltration with a centrifugal filter (300,000 MWCO) to remove free IL-12. After filtration through a 0.22 µm syringe-driven filter membrane, the size distribution of IL-12/m was characterized by dynamic laser scattering (DLS), and the surface charge was determined by zeta potential measurement (Zetasizer Nano-ZS, Malvern, U.K.). To obtain a TEM photograph, IL-12/m was first dialyzed overnight against DI water (100,000 MWCO) to remove phosphate and NaCl, and then stained with phosphotungstic acid (PTA) (2%, w/v) and placed on a 400 mesh copper grid for TEM observation (JEM-1400, JEOL).

The results obtained are shown in Figures 5 and 6. As can be seen from Figures 5 and 6, the prepared micelles are 40 nm size particles whose surface potential has been neutralized and which encapsulate IL-12 at all possible concentrations with high protein encapsulation efficiency.

### 3. In vitro stability and pH-controlled drug release profile (Figure 7)

To measure the *in vitro* stability of IL-12/m, a micelle sample was prepared and concentrated to contain 100 µg/mL PEG-p(Lys-CDM) in PBS buffer of pH 7.4. Then, the micelle solution was diluted 10-fold in PBS buffers of pH 7.4 and pH 6.5 in the respective tubes, and the diluted solutions were monitored at given time points by DLS techniques to record changes in the derivative count rate, average size and polydispersity of micelles. In a drug release test, IL-12/m samples each containing 10 µg of Alexa-647-labeled IL-12 in 200 µL of PBS (pH 7.4) were each dialyzed in a dialysis cassette (100,000 MWCO) at room temperature against 1 L of PBS of pH 6.5 or pH 7.4. The samples were taken at given time points from the cassette and measured for their fluorescence intensity with a nanodrop spectrometer to calculate the cumulative release ratio.

The results obtained are shown in Figure 7. As can be seen from Figure 7, the prepared micelles are stable at pH 7.4, whereas they are unstable at pH6.5.

### 4. In vitro biological activity assay (Figure 8)

In an *in vitro* biological activity assay, spleen cells (9 weeks) collected from BALB/c mice were seeded in 96-well plates (10⁵ cells/well) containing 100 µL per well of RPMI medium (containing 10% FBS, 1 × penicillin-streptomycin, 2 mM L-glutamine and 50 µM 2-mercaptoethanol). Released IL-12 was prepared as follows: 10 µg/mL IL-12/m was diluted 10-fold in PBS of pH 5.0 and incubated overnight, and then further diluted to determine the IL-12 equivalent concentration in PBS buffer of pH 7.4. Then, the cells were incubated for 24 hours with any of native IL-12, released IL-12 or IL-12/m (0 to 100 ng/mL IL-12 equivalent concentrations). After centrifugation at 500 g × 5 minutes, the culture supernatants were collected and the concentration of IFN-γ contained therein was measured with an ELISA kit.

The results obtained are shown in Figure 8. As can be seen from Figure 8, IL-12 released from the micelles retains its activity.

### 5. Preparation of lyophilized IL-12/m (Figure 9 and Table 1)

For lyophilization, IL-12/m was first prepared and purified according to the above protocol, and the final purified solution was supplemented with trehalose (5% of the total weight of the IL-12/m solution) as a lyoprotectant. This mixture was frozen with liquid nitrogen and lyophilized. The lyophilized powder was reconstituted by adding DI water to reach the initial solution volume, and then allowed to stand at 4°C for 5 hours. The reconstituted IL-12/m was loaded on a Zetasizer for DLS and zeta potential measurement, and the concentration of free IL-12 in the IL-12/m solution was detected by ELISA.

To measure the biological activity of lyophilized IL-12/m, the *in vitro* assay using spleen cells was used again. After reconstitution, lyophilized IL-12/m and released IL-12 were prepared according to the previously reported protocols. For comparison, non-lyophilized original IL-12/m was prepared. These samples were each diluted to a given equivalent concentration (0.1 to 10 ng/mL) and incubated for 24 hours with spleen cells. Then, the supernatants were collected for ELISA assay and measured for their IFN-γ concentration.

The results obtained are shown in Figure 9 and Table 1. As can be seen from Figure 9 and Table 1, the lyophilized micelles have the same physicochemical properties and activity as the micelles before lyophilization.

**Table 1. Properties of original and lyophilized IL-12/m**

| | Average size¹ (nm) | PDI | Zeta potential (mV) |
|---|---|---|---|
| IL-12 | 14.7 ± 1.4 | 0.30 ± 0.03 | -28.5 ± 3.2 |
| Original IL-12/m | 42.2 ± 1.7 | 0.23 ± 0.02 | -4.1 ± 1.0 |
| Lyophilized IL-12/m | 46.6 ± 1.2 | 0.28 ± 0.02 | -2.9 ± 0.7 |

| | | | |
|---|---|---|---|
| ¹Determined by DLS | | | |

### 6. Blood circulation (Figure 10)

Blood circulation profiles were studied under both conditions of high dose (10 µg IL-12 equivalence) and low dose (1 µg IL-12 equivalence). Corresponding drugs were each dispersed in 200 µL of PBS and intravenously (i.v.) injected through the mouse tail vein. After injection, mouse blood samples (n = 3 per group) were taken from the abdominal aorta at given time points and stored in heparinized tubes. Then, the blood samples were centrifuged at 1000 g for 10 minutes, and their supernatants were collected as plasma samples. The plasma samples were each diluted (in PBS of pH 7.4) to be within an appropriate concentration range for ELISA assay, and the concentration of released IL-12 was measured.

To measure the total amount of IL-12 delivered by the micelles, the samples were first diluted 5-fold in PBS of pH 5.0 to disrupt the micelles, and incubated overnight at 4°C, and then further diluted in the dilution buffer attached to an ELISA kit to be within an appropriate concentration range for ELISA assay. The endogenous IL-12 level in PBS-injected mice was not detectable with the ELISA kit. A monophasic attenuation model was assumed for prediction of the plasma half-life, and the area under the curve (AUC) was calculated with GraphPad Prism software.

The results obtained are shown in Figure 10. As can be seen from Figure 10, micellization enhances the blood retention of IL-12 and allows suppression of IL-12 release in blood.

### 7. In vivo confocal laser scanning microscope (Figure 11)

The circulation status of IL-12/m was followed up by *in vivo* confocal laser scanning microscope (IVCLSM) observation on the right earlobe of C57BL/6 mice at 8 weeks of age. The mice were anesthetized with 2.5% isoflurane and fixed. 10 µg of Alexa 647-IL-12 or Alexa 647-IL-12/m was dispersed in 100 µL of PBS and intravenously injected into each mouse. Continuous IVCLSM observation was initiated immediately after injection and continued for 3 hours. After 3 hours, tissue samples were taken from the mice. The tissue samples were observed under an IVCLSM to investigate the microdistribution of fluorescence.

The results obtained are shown in Figure 11. As can be seen from Figure 11, IL-12 alone causes transfer to normal tissues which is responsible for side effects, whereas IL-12/m allows suppression of IL-12 extravasation.

### 8. In vivo distribution (Figures 12 to 14)

*In vivo* distribution profiles were studied under both conditions of high dose (10 µg IL-12 equivalence) and low dose (1 µg IL-12 equivalence). Experiments were performed on mice bearing melanoma and TBNC tumor with an average volume of 200 mm³. The drugs to be injected were each dispersed in 200 µL of PBS.

As to a high dose experiment, Alexa Fluor-647-labeled IL-12 was first used in melanoma-bearing mice for fluorescence-based quantification. 10 µg of IL-12 or IL-12/m equivalence was intravenously injected. At 24 hours after injection, tissue samples (heart, liver, spleen, lung, kidney and tumor) were taken from the mice. The tissue samples were first imaged with an IVIS imaging system (PerkinElmer, USA) and then homogenized in PBS of pH 7.4 with a Dounce tissue homogenizer. After centrifugation at 2000 g × 15 minutes, the supernatants were collected and quantified for fluorescence intensity via a multi-well plate reader (Tecan, Switzerland). Further, to directly quantify IL-12 distribution, non-labeled IL-12 and IL-12/m were intravenously injected into TNBC tumor-bearing mice. At 24 hours after injection, tissue samples were collected. For protein extraction in a tissue extraction reagent containing a proteinase inhibitor, the tissues were homogenized. The sample extracts were centrifuged at 2000 g × 15 minutes to collect their supernatants. The concentrations of released IL-12 and total IL-12 in the supernatants were detected by ELISA after a corresponding dilution protocol.

As to a low dose experiment, melanoma-bearing mice were intravenously injected with non-labeled IL-12 or IL-12/m (1 µg IL-12 equivalence per injection). At given time points (at 1, 4, 8, 24 and 48 hours after injection), tissue samples were taken from the mice. The samples were separated and measured by ELISA according to the same protocol as used in the high dose experiment.

The results obtained are shown in Figures 12 to 14. As can be seen from Figures 12 to 14, IL-12/m shows higher tumor accumulation than IL-12 alone. Moreover, IL-12/m accumulation was observed in the liver and other normal tissues, but the released amount of IL-12 was limited because of normal pH within these tissues.

### 9. Systemic and intratumoral cytokine secretion (Figures 15 and 16)

Systemic and intratumoral IFN-γ and IL-10 levels after treatment were detected to evaluate systemic and intratumoral immune responses. Moreover, experiments were performed under both conditions of high dose (10 µg IL-12 equivalence) and low dose (1 µg IL-12 equivalence).

As to a high dose experiment, TNBC tumor-bearing mice were i.v. injected with 10 µg of IL-12 or IL-12/m equivalence on Day 0 and Day 3. 100 µL of blood was collected every day from the orbital vein with a heparinized capillary tube, and then centrifuged at 1000 g × 10 minutes to separate a plasma sample (samples on Day 0 and Day 3 were collected before injection of IL-12 or IL-12/m). On Day 7, tumors were taken from the mice. Then, the tumors were homogenized with a Dounce homogenizer in a tissue protein extraction buffer containing a proteinase inhibitor. After centrifugation at 2000 g × 15 minutes, the supernatants were collected. The concentrations of IFN-γ and IL-10 in the plasma and tumor samples were measured with an ELISA kit.

As to a low dose experiment, melanoma-bearing mice were i.v. injected with 1 µg of IL-12 or IL-12/m equivalence on Day 0. On Days 1, 2 and 3, blood, spleen and tumor samples were taken from the mice. The blood samples were centrifuged at 1000 g × 10 minutes to separate plasma. The tumors and spleens were each homogenized with a Dounce homogenizer in a tissue protein extraction buffer containing a proteinase inhibitor. After centrifugation at 2000 g × 15 minutes, the supernatants were collected. The concentrations of IFN-γ and IL-10 in the samples were measured with an ELISA kit.

The results obtained are shown in Figures 15 and 16. As can be seen from Figures 15 and 16, IL-12 alone causes an increase in the expression level of IL-10 which is an anti-inflammatory cytokine, whereas IL-12/m causes an increase in the expression level of an inflammatory cytokine.

### 10. Systemic toxicity (Figures 17 and 18)

The systemic toxicity of IL-12 and IL-12/m was evaluated according to the same dose schedule as used in the high dose experiment for systemic and intratumoral cytokine secretion. Tumor-free Balb/c mice at 5 weeks of age were i.v. injected with 10 µg equivalent of IL-12 or IL-12/m on Day 0 and Day 3. A body weight change curve was plotted against time, and the body weight of each mouse was monitored individually. On Day 7, blood and tissue samples were taken from the mice. The blood was centrifuged at 1000 g × 10 minutes to separate plasma. The plasma concentrations of total protein (TP), alanine transaminase (ALT), blood urea nitrogen (BUN) and lipase were detected with a DRI-CHEM NX500 blood analyzer (Fujifilm, Japan). TP and ALT were used for hepatopathy evaluation. BUN was used for nephropathy evaluation, while lipase was used for pancreatopathy evaluation. Fresh liver and kidney samples were taken, and the tissue sections thereof were stained with H&E.

The results obtained are shown in Figures 17 and 18. As can be seen from Figures 17 and 18, IL-12 alone shows toxicity to the liver, pancreas and kidney, whereas micellization allows a reduction in toxicity.

### 11. Treatment of mice (Figures 19 to 24)

In an antitumor activity experiment, different dose schedules were used. IL-12 or IL-12/m was dispersed in 200 µL of PBS and intravenously injected into mice through the tail vein. 100 µg of an anti-PD-1 antibody was dissolved in 100 µL of PBS and intraperitoneally (i.p.) injected into the right hypogastric region of each mouse.

The results obtained are shown in Figures 19 to 24. As can be seen from Figures 19 to 24, IL-12/m has higher antitumor activity than IL-12 alone, and enhances its therapeutic effect when used in combination with an immune checkpoint inhibitor. Moreover, IL-12/m shows the same level of safety as in the untreated group.

### 12. Histology (Figures 25 to 28)

Fresh tumor and organ samples were taken from mice, and specimens of tissue sections were prepared. In the case of lung samples, lungs were first soaked overnight in 4% paraformaldehyde (PFA) and then soaked in a 30% sucrose solution until the samples completely sank to the bottom. Subsequently, the lung samples were washed twice with an optimal cutting temperature compound (O.C.T. compound) to remove the sucrose solution, and were completely soaked in the O.C.T. compound and then rapidly frozen in liquid nitrogen. In the case of tumor and other organs, fresh samples were directly soaked in the O.C.T. compound and frozen in liquid nitrogen. The frozen tissue samples were sliced into 10 µm sections at -30°C in a cryostat (Leica, German).

For immunohistochemical (IHC) staining, the fresh tissue sections were first washed with PBS to remove the O.C.T compound, and then fixed with 4% PFA for 5 minutes. Nuclei were stained for 15 minutes using a 100-fold diluted Hoechst 33342 solution, followed by antibody staining. For staining of membrane antigens (CD8, NKg2D and PD-L1), their antibodies were diluted 50-fold in PBS for use as working solutions, incubated for 30 minutes and then added dropwise onto the tissue sections.

For staining of intracellular antigens (Tbet and Foxp3), the sections were first made permeable with a Foxp3/transcription factor staining buffer set according to the protocol provided by the manufacturer, and then stained with their antibodies using the same procedures as above. After staining, the sections were washed with DI water for 3 × 5 minutes, dried and mounted in a mounting agent. The sections were observed under a CLSM (LSM-700, Zeiss, German) with a 10x ocular lens. Fluorescence intensity from the labeled antibody in each file was measured by ZEN lite software (v3.3, Zeiss, German), and the number of antibody-positive cells was calculated by dividing the total intensity by the average fluorescence intensity of 10 individual positive cells.

For histopathological examination, H&E staining was conducted. Tissue sections were prepared through the same protocol as described above. The sections were stained with an H&E staining kit according to the manufacturer's instructions. The sections were observed under an optical microscope (Zeiss, German).

The results obtained are shown in Figures 25 to 28. As can be seen from Figures 25 to 28, killer T cells, helper T cells and NK cells enhanced their infiltration ability after administration of IL-12/m, whereas an increase in regulatory T cells was suppressed after administration of IL-12/m. Moreover, the expression level of PD-L1 was increased after administration of IL-12/m, thus elucidating the mechanism of synergy with an anti-PD-1 antibody.

### 13. Flow cytometry (Figure 29)

For quantitative analysis of intratumoral lymphocyte infiltration, a flow cytometry experiment was performed. For preparation of samples, fresh tumors were taken from mice. The tumors were homogenized in PBS supplemented with 2% FBS (running buffer), and RBCs were then removed through RBC lysis buffer to thereby prepare single cell suspensions. The cells were suspended again in the running buffer and filtered through a 100 µm cell strainer. The suspensions were adjusted to a cell density of 10⁶ cells/mL and provided for antibody staining in the running buffer. The amount of each antibody required for staining was calculated according to the manufacturer's instructions. The cells were incubated together with each antibody for 30 minutes, and the running buffer was used for centrifugal washing at 500 g × 5 minutes and subsequent re-suspension, which were repeated twice. The samples were finally adjusted to 10⁶ cells/mL and loaded on a flow cytometer (LSR II, BD Bioscience, USA).

The results obtained are shown in Figure 29. As can be seen from Figure 29, various T cells including killer T cells and helper T cells are shown to infiltrate into tumors after administration of IL-12/m.

## Claims

1. A polymeric complex comprising a cytokine and a block copolymer represented by the following formula (1): 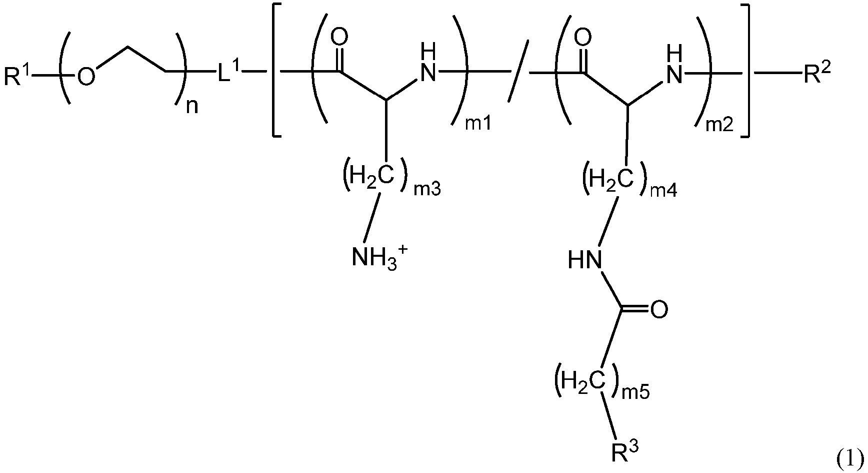
[wherein R¹ and R² each independently represent a hydrogen atom, or an optionally substituted linear or branched alkyl group containing 1 to 12 carbon atoms, or an azide,
an amine, maleimide, a ligand or a labeling agent,
R³ represents a compound represented by the following formula (I): 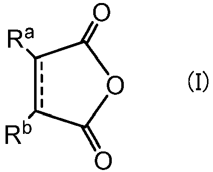 (wherein R^{a} and R^{b} each independently represent a hydrogen atom, or an optionally substituted alkyl group, an alkenyl group, a cycloalkyl group, an aryl group, an aralkyl group, an acyl group, a heterocyclic group, a heterocyclic alkyl group, a hydroxy group, an alkoxy group or an aryloxy group. Alternatively, R^{a} and R^{b} may be joined with each other to form an aromatic ring or a cycloalkyl ring together with the carbon atoms to which they are attached respectively. The bond between the carbon atoms to which R^{a} and R^{b} are attached respectively may be a single bond or a double bond),
L¹ represents NH, CO, or a group represented by the following formula (11):
-(CH₂)ₚ₁-NH- (11)
(wherein p1 represents an integer of 1 to 6), or
a group represented by the following formula (12):
-L^{2a}-(CH₂)_{q1}-L^{3a}- (12)
(wherein L^{2a} represents OCO, OCONH, NHCO, NHCOO, NHCONH, CONH or COO, L^{3a} represents NH or CO, and q1 represents an integer of 1 to 6),
m1 and m2 each independently represent an integer of 0 to 500 (provided that the sum of m1 and m2 represents an integer of 10 to 500), m3, m4 and m5 each independently represent an integer of 1 to 5, and n represents an integer of 0 to 500, and
the symbol "/" means that (m1 + m2) units of the respective monomer units shown on the left and right sides of this symbol may be in any sequence].

2. The complex according to claim 1, wherein the compound represented by formula (I) is at least one of compounds represented by the following formulae (Ia) to (Ig).

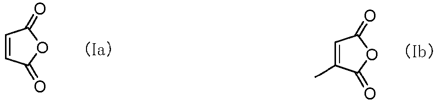

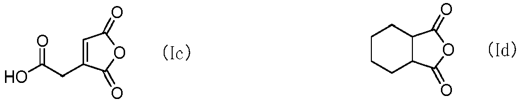

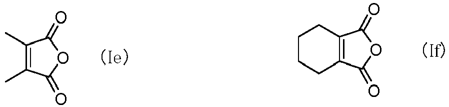

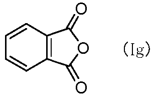

3. The complex according to claim 2, wherein the compound represented by formula (I) is a compound represented by the following formula (Ia) or (Ib).

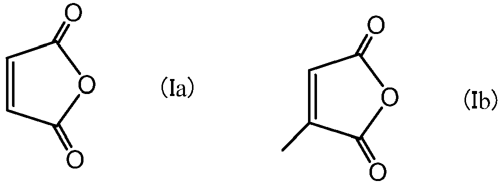

4. The complex according to claim 1, wherein the block copolymer represented by formula 1 is a block copolymer represented by the following formula (2).

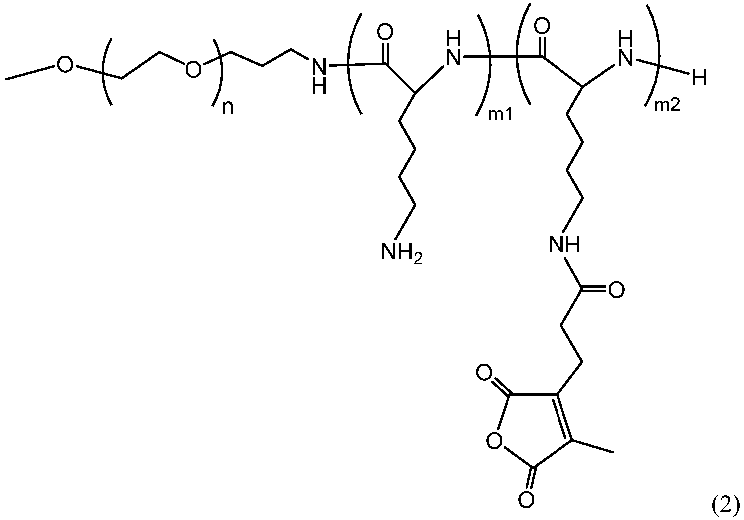

5. The complex according to claim 1, wherein the cytokine is covalently bonded to the block copolymer represented by formula 1.

6. The complex according to claim 5, wherein the covalent bond is cleaved in a pH-dependent manner.

7. The complex according to claim 1, wherein the cytokine is interleukin 12.

8. A cytokine delivery device comprising the polymeric complex according to any one of claims 1 to 7 for use in cytokine delivery to any site selected from a cell surface site, an intracellular site and an extracellular site.

9. A cytokine delivery kit comprising a block copolymer represented by the following formula (1) for use in cytokine delivery to any site selected from a cell surface site, an intracellular site and an extracellular site: 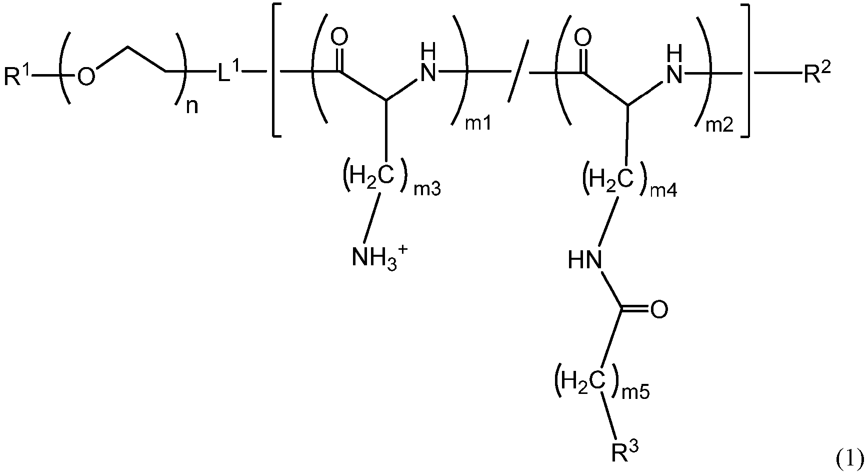
[wherein R¹ and R² each independently represent a hydrogen atom, or an optionally substituted linear or branched alkyl group containing 1 to 12 carbon atoms, or an azide, an amine, maleimide, a ligand or a labeling agent,
R³ represents a compound represented by the following formula (I): 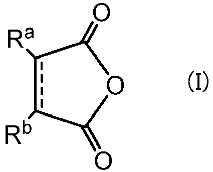 (wherein R^{a} and R^{b} each independently represent a hydrogen atom, or an optionally substituted alkyl group, an alkenyl group, a cycloalkyl group, an aryl group, an aralkyl group, an acyl group, a heterocyclic group, a heterocyclic alkyl group, a hydroxy group, an alkoxy group or an aryloxy group. Alternatively, R^{a} and R^{b} may be joined with each other to form an aromatic ring or a cycloalkyl ring together with the carbon atoms to which they are attached respectively. The bond between the carbon atoms to which R^{a} and R^{b} are attached respectively may be a single bond or a double bond),
L¹ represents NH, CO, or a group represented by the following formula (11):
-(CH₂)ₚ₁-NH- (11)
(wherein p1 represents an integer of 1 to 6), or
a group represented by the following formula (12):
-L^{2a}-(CH₂)_{q1}-L^{3a}- (12)
(wherein L^{2a} represents OCO, OCONH, NHCO, NHCOO, NHCONH, CONH or COO,
L^{3a} represents NH or CO, and q1 represents an integer of 1 to 6),
m1 and m2 each independently represent an integer of 0 to 500 (provided that the sum of m1 and m2 represents an integer of 10 to 500), m3, m4 and m5 each independently represent an integer of 1 to 5, and n represents an integer of 0 to 500, and
the symbol "/" means that (m1 + m2) units of the respective monomer units shown on the left and right sides of this symbol may be in any sequence].

10. The kit according to claim 9, wherein the compound represented by formula (I) is at least one of compounds represented by the following formulae (Ia) to (Ig).

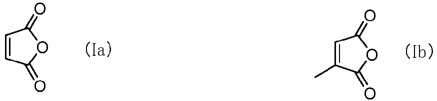

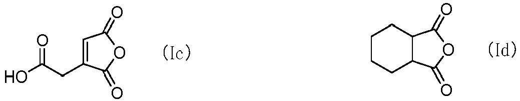

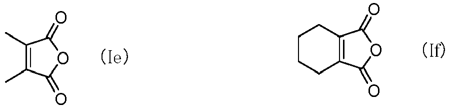

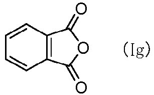

11. The kit according to claim 9, wherein the compound represented by formula (I) is a compound represented by the following formula (Ia) or (Ib).

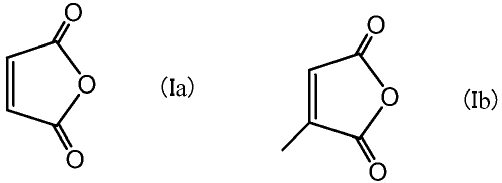

12. The kit according to claim 9, wherein the block copolymer represented by formula 1 is a block copolymer represented by the following formula (2).

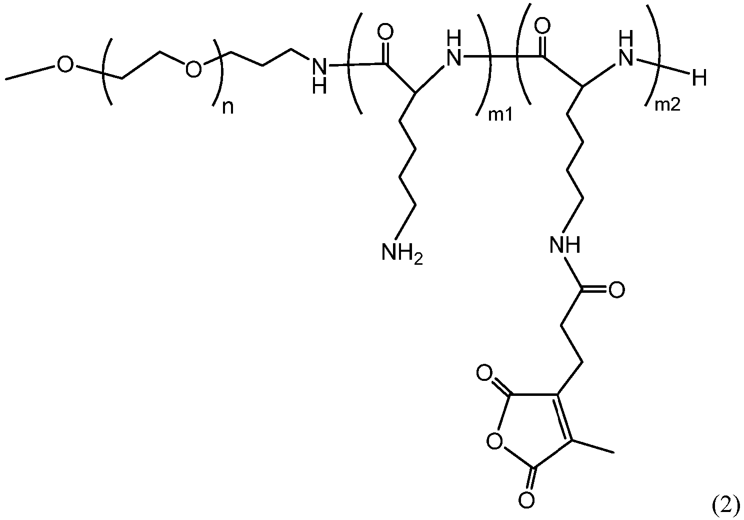

13. The kit according to any one of claims 9 to 12, wherein the cytokine is interleukin 12.

14. A pharmaceutical composition comprising the polymeric complex according to any one of claims 1 to 7.

15. The pharmaceutical composition according to claim 14 for use in immunotherapy or antitumor therapy.
